(19) Europäisches Patentamt / European Patent Office / Office européen des brevets

(11) **EP 2 108 327 B1**

(12) **EUROPEAN PATENT SPECIFICATION**

(45) Date of publication and mention
of the grant of the patent:
**25.07.2018 Bulletin 2018/30**

(21) Application number: **07830681.8**

(22) Date of filing: **26.10.2007**

(51) Int Cl.:
*A61B 34/30* *(2016.01)*     *A61B 34/37* *(2016.01)*
*A61B 34/35* *(2016.01)*     *A61B 34/00* *(2016.01)*
*A61B 18/14* *(2006.01)*     *A61B 1/018* *(2006.01)*
*A61B 90/00* *(2016.01)*     *A61B 34/20* *(2016.01)*
*A61B 34/10* *(2016.01)*

(86) International application number:
**PCT/JP2007/070947**

(87) International publication number:
**WO 2008/093455 (07.08.2008 Gazette 2008/32)**

(54) **ENDOSCOPIC OPERATION DEVICE**

ENDOSKOPISCHES OPERATIONSGERÄT

DISPOSITIF D'OPÉRATION ENDOSCOPIQUE

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **01.02.2007 JP 2007023302**

(43) Date of publication of application:
**14.10.2009 Bulletin 2009/42**

(73) Proprietor: **Olympus Corporation
Tokyo 192-8507 (JP)**

(72) Inventors:
• **SUGIYAMA, Yuta
Tokyo 192-8512 (JP)**

• **TAKAHASHI, Kazuhiko
Tokyo 192-8512 (JP)**

(74) Representative: **von Hellfeld, Axel
Wuesthoff & Wuesthoff
Patentanwälte PartG mbB
Schweigerstrasse 2
81541 München (DE)**

(56) References cited:
EP-A2- 0 776 739     JP-A- 05 261 692
JP-A- 2004 180 781     JP-A- 2005 131 417
JP-A- 2006 271 546     US-A1- 2004 147 920
US-A1- 2005 096 502     US-A1- 2006 142 657
US-A1- 2006 142 824     US-B2- 7 035 716

**Description**

Technical Field

The present invention relates to an endoscopic surgical apparatus wherein treatment is performed under an endoscopic observation using an accessory inserted into a cavity in the body through a channel of the endoscope.

Background Art

In an endoscopic surgical system disclosed in Jpn. Pat. Appln. KOKAI Publication No. 5-42167, an accessory is inserted into a cavity in the body through a channel of an endoscope and treatment such as an ablation of a lesion part is performed in the cavity in the body using the accessory under an endoscopic observation. In such accessory, a degree of freedom in operation of the distal end portion of the accessory is few and the distal end portion of the accessory is moved mainly by operating the endoscope itself. Therefore, it is examined that an accessory is made to be a multi-joint type and further an electric control type to improve an operability of the accessory in order to enable to make the distal end portion of the accessory move just as an operator intends.
On the other hand, other than an endoscopic surgical system wherein an endoscope is used, various electric controls are performed. In a master-slave type of robotic surgical system disclosed in Jpn. Pat. Appln. KOKAI Publication No. 2004-223128, a slave manipulator is driven following a master manipulator and treatment is performed by a medical instrument on the distal end portion of the slave manipulator. When the medical instrument is approached to a boundary of a movement range more than a certain warning amount, a minimum value necessary for an operation input to a master manipulator is increased, and therefore, it is possible to recognize whether the medical instrument is placed within the movement range or not. In a laser scalpel treatment system disclosed in Jpn. Pat. Appln, KOKAI Publication No. 9-131351, treatment is performed by radiating a laser beam to a treatment part. An observation image of the treatment part is obtained by CCD camera placed outside of a patient, a radiation range of the laser beam is set on the observation image, and the radiation of the laser beam is forced to be stopped when a radiation position of the laser beam is placed outside of the range.
US 2006/0142657 A1 relates to a surgical system for orthopedic joint replacement. The surgical system includes a haptic device with an arm. The arm includes position sensors for determining a position or orientation of the arm. The haptic device say surgical device configured to be manipulated by a user to move a surgical tool to perform a procedure on a patient. A haptic object configured for assisting the user in guiding the tool to the surgical side is represented graphically as a funnel shaped volume by haptic forces reflected to the user. The haptic device senses a velocity and/or acceleration of the tool and applies an appropriate corresponding wrench.

Disclosure of Invention

When treatment is performed in a cavity in the body by an endoscopic surgical system, information which can be referred to is only an observation image obtained by an endoscope, it is difficult to, for example, instantly recognize a three-dimensionally positional relationship between a lesion part and an accessory and adequately recognize force applied between a lesion part and an accessory, and therefore, it is difficult for an operator except for a skilled one to accurately perform treatment.
The present invention has been made in view of the above-mentioned problem, and is directed to provide an endoscopic surgical apparatus capable of improving accuracy of treatment.
The problem is solved by an endoscopic surgical apparatus according to claim 1.
In an aspect of the present disclosure an endoscopic surgical apparatus comprises: a treatment accessory configured to be inserted through a channel of an endoscope, including a treatment portion with a treatment function, and having a movement function; a setting means configured to set a reference position as a reference in treatment by the treatment accessory and a reference direction relative to the reference position; a detecting means configured to detect a movement state of the treatment accessory in the reference direction relative to the reference position; a control means configured to control the movement function or the treatment function on the basis of the movement state.
In this aspect, the movement function of the treatment accessory or the treatment function of the treatment portion is controlled on the basis of the movement state of the treatment accessory in the reference direction relative to the reference position. Therefore, it is possible to improve accuracy of treatment.
In a preferred aspect of the present disclosure, the control means is configured to vary the movement state of the treatment accessory in the reference direction relative to the reference position or a treatment capability of the treatment portion, according to the movement state.
In this preferred aspect, the movement state of the treatment accessory in the reference direction relative to the reference position or the treatment capability of the treatment portion is varied on the basis of the movement state, whereby enabling to prevent unnecessary treatment. Therefore, it is possible to further improve the accuracy of the treatment and to increase speed of treatment since an operator can perform treatment with a sense of safety.

In a preferred aspect of the present disclosure, the setting means is configured to set a plurality of reference directions, variations of the movement state or variations of the treatment capability with respect to the movement state for the plurality of reference directions are different from each other.

In this preferred aspect, the variations of the movement state or the variations of the treatment capability relative to the movement state are different from each other corresponding to the reference directions relative to the reference position. Therefore, the accessory can be controlled more properly and it is possible to further improve the accuracy of the treatment and increase the speed of the treatment.

In a preferred aspect of the present disclosure, the setting means includes a setting accessory with a setting portion for setting the reference position and is configured to set the reference position on a position where the setting portion is placed.

In this preferred aspect, the reference position is set on the position where the setting portion of the setting accessory is placed. Therefore, the reference position can be set correctly and rapidly and it is possible to further improve the accuracy of the treatment and increase the speed of the treatment.

In an aspect of the present disclosure, an endoscopic surgical apparatus comprises: a treatment accessory configured to be inserted through a channel of an endoscope, including a treatment portion with a treatment function, and having a movement function; a setting means configured to set a reference position as a reference in treatment by the treatment accessory and a reference direction relative to the reference position; a detecting means configured to detect a movement state of the treatment accessory in the reference direction relative to the reference position; a warning means configured to generate a warning; and a control means configured to make the warning means generate a warning on the basis of the movement state.

In this aspect, a warning is generated on the basis of the movement state of the treatment accessory in the reference direction relative to the reference position. Therefore, it is possible to improve accuracy of treatment and to increase speed of treatment since an actuation of the treatment accessory is not limited directly.

In an aspect of the present disclosure, an endoscopic surgical apparatus comprises: a treatment accessory configured to be inserted through a channel of an endoscope, including a treatment portion with a treatment function, and having a movement function; a setting means configured to set a reference position as a reference in treatment by the treatment accessory and a reference direction relative to the reference position; a detecting means configured to detect a movement state of the treatment accessory in the reference direction relative to the reference position; an operating means configured to operate the movement function or the treatment function of the treatment accessory; and a control means for controlling a function of the operating means on the basis of the movement state of the treatment accessory detected by the detecting means.

In this aspect, the function of the operating means for operating the movement function or the treatment function of the treatment accessory is controlled on the basis of the movement state of the treatment accessory in the reference direction relative to the reference position. Therefore, it is possible to improve accuracy of treatment.

In an aspect of the present disclosure, an endoscopic surgical apparatus comprises: a treatment accessory configured to be inserted through a channel of an endoscope, including a treatment portion with a treatment function, and having a movement function; a setting means configured to set a reference position as a reference in treatment by the treatment accessory and a following condition of the treatment accessory relative to the reference position; and a control means configured to control the movement function on the basis of the reference position and the following condition.

In this aspect, the movement function of the treatment accessory is controlled on the basis of the reference position and the following condition of the treatment accessory relative to the reference position. Therefore, it is possible to improve accuracy of treatment and increase speed of treatment, in particular, in the case where the treatment accessory can not be observed by the endoscope.

In an aspect of the present disclosure, an endoscopic surgical apparatus comprises: a treatment accessory configured to be inserted through a channel of an endoscope, including a treatment portion with a treatment function, and having a movement function; a setting means configured to set a reference position as a reference in treatment by the treatment accessory; a control means configured to control the movement function such that the treatment accessory is moved toward the reference position.

In this aspect, the movement function of the treatment accessory is controlled such that the treatment accessory is moved toward the reference position. Therefore, it is possible to improve accuracy of treatment and increase speed of treatment, in particular, in the case where the treatment accessory can not be observed by the endoscope.

Brief Description of Drawings

FIG. 1 is a schematic view showing an endoscopic surgical apparatus according a first embodiment of the present invention;

FIG. 2 is a block diagram showing the endoscopic surgical apparatus according to the first embodiment of the present invention;

FIG. 3 is a schematic view showing an endoscope system according to the first embodiment of the present invention;

FIG. 4 is a view for explaining a method for using the endoscopic surgical apparatus according to the first embodiment of the present invention;

FIG. 5 is a flow chart showing a movement function control of the endoscopic surgical apparatus according to the first embodiment of the present invention;

FIG. 6 is a flow chart showing a treatment function control of the endoscopic surgical apparatus according to the first embodiment of the present invention;

FIG. 7 is a view showing a graph of a velocity transformation variable $\alpha$ in the movement function control of the endoscopic surgical apparatus according to the first embodiment of the present invention;

FIG. 8 is a view showing a graph of a velocity transformation variable $\beta$ in the movement function control of the endoscopic surgical apparatus according to the first embodiment of the present invention;

FIG. 9 is a view showing a graph of an output transformation variable $\gamma$ in the treatment function control of the endoscopic surgical apparatus according to the first embodiment of the present invention;

FIG. 10 is a view showing a graph of an output transformation variable $\sigma$ of the treatment function control of the endoscopic surgical apparatus according to the first embodiment of the present invention;

FIG. 11 is a view showing a graph of an output transformation variable $\omega$ in the treatment function control of the endoscopic surgical apparatus according to the first embodiment of the present invention;

FIG. 12 is a schematic view showing a plurality of reference directions in a function control of an endoscopic surgical apparatus according to a second embodiment of the present invention;

FIG. 13 is a view showing a graph of a first velocity transformation variable $\alpha1$ for a first reference direction in a movement function control of the endoscopic surgical apparatus according to the second embodiment of the present invention;

FIG. 14 is a view showing a graph of a second velocity transformation variable $\alpha2$ for a second reference direction in the movement function control of the endoscopic surgical apparatus according to the second embodiment of the present invention;

FIG. 15 is a view showing a graph of a first output transformation variable $\gamma1$ for the first reference direction in a treatment function control of the endoscopic surgical apparatus according to the second embodiment of the present invention;

FIG. 16 is a view showing a graph of a second output transformation variable y2 for the second reference direction in the treatment function control of the endoscopic surgical apparatus according to the second embodiment of the present invention;

FIG. 17 is a schematic view showing an endoscope system according to a third embodiment of the present invention;

FIG. 18 is a block diagram showing an endoscopic surgical apparatus according to the third embodiment of the present invention;

FIG. 19 is a view for explaining a method for using the endoscopic surgical apparatus according to the third embodiment of the present invention;

FIG. 20 is a flow chart showing a movement function control of the endoscopic surgical apparatus according to the third embodiment of the present invention;

FIG. 21 is a view for explaining a method for using an endoscopic surgical apparatus according to a modified example of the third embodiment of the present invention;

FIG. 22 is a perspective view showing a setting portion of a robotic accessory according to a fourth embodiment not according to the present invention;

FIG. 23 is a view for explaining a method for using an endoscopic surgical apparatus according to the fourth embodiment not according to the present invention;

FIG. 24 is a perspective view showing a setting portion of the robotic accessory according to a modified example of the fourth embodiment not according to the present invention;

FIG. 25 is a schematic view showing an endoscope system according to a fifth embodiment not according to the present invention;

FIG. 26 is a flow chart showing a warning control of an endoscopic surgical apparatus according to the fifth embodiment not according to the present invention;

FIG. 27 is a schematic view showing an endoscope system according to a sixth embodiment not according to the present invention;

FIG. 28 is a block diagram showing an endoscopic surgical apparatus according to the sixth embodiment not according to the present invention;

FIG. 29 is a flow chart showing an active control of the endoscopic surgical apparatus according to the sixth embodiment not according to the present invention;

FIG. 30 is a view for explaining a method for using an endoscopic surgical apparatus and showing a state at a start of a following control, according to a seventh embodiment not according to the present invention;

FIG. 31 is a view for explaining the method for using the endoscopic surgical apparatus and showing the following control, according to the seventh embodiment not according to the present invention;

FIG. 32 is a flow chart showing the following control of the endoscopic surgical apparatus according to the seventh embodiment not according to the present invention;

FIG. 33 is a view for explaining a method for using an endoscopic surgical apparatus according to an eighth embodiment not according to the present invention; and

FIG. 34 is a flow chart showing an automatic movement control of the endoscopic surgical apparatus according to the eighth embodiment not according to the present invention.

Best Mode for Carrying Out the Invention

Hereinafter, each embodiment of the present invention will be explained referring to the drawings.

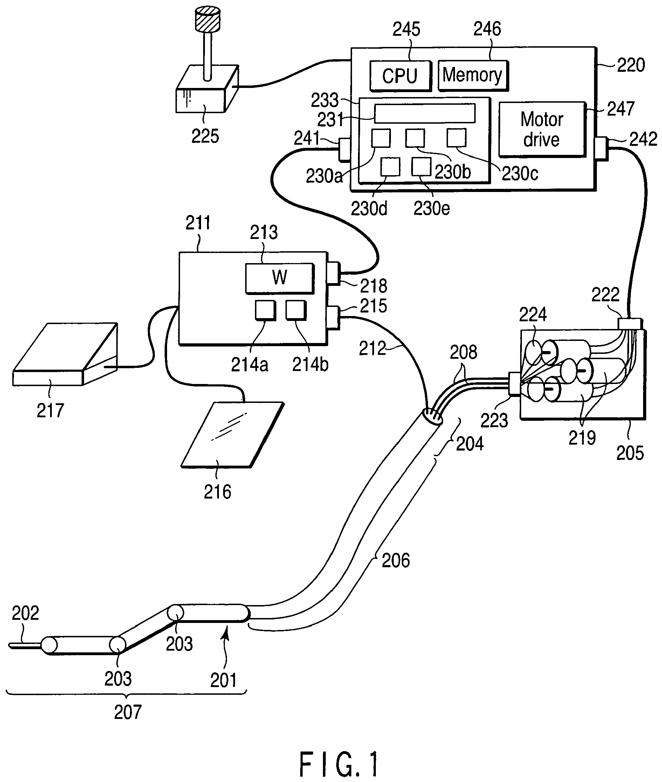

FIGS. 1 to 11 show a first embodiment of the present invention.

Referring to FIG. 1, an endoscopic surgical apparatus according to the embodiment includes a robotic accessory 201 as a treatment accessory with a movement function and a treatment function. The movement function and the treatment function of the robotic accessory 201 is configured to be controlled on the basis of manipulation by an operator in a normal mode while on the basis of manipulation by an operator and set control parameters in a movement function control mode or a treatment function control mode. The robotic accessory 201 also functions as a setting accessory for setting a reference point as a reference position which is one of the control parameters and is configured to set the reference point on the position where the distal end of the robotic accessory 201 as a setting portion is placed.

Regarding a structure related to the movement function of the robotic accessory 201, the robotic accessory 201 is a master-slave type of multi-joint electric accessory configured to be actuated following manipulation to a joystick 225.

That is, the joystick 225 for inputting a target position and a target attitude of a movement of the robotic accessory 201 is connected to a robotic accessory control apparatus 220 as a control means. It is noted that it is possible to use a haptic device, a touch panel, voice recognition and so on other than the joystick 225 as a movement instruction input means for operating a movement of the robotic accessory 201. The robotic accessory control apparatus 220 includes CPU 245 configured to perform various kinds of arithmetic process, a memory 246 configured to storage various kinds of data obtained by communication, arithmetic, input and the like, and a motor drive 247 configured to control a motor box 205. A communicating portion for the motor box 242 of the robotic accessory control apparatus 220 is connected to a motor box communicating portion 222 of the motor box 205, and communication is performed between the robotic accessory control apparatus 220 and the motor box 205.

Various kinds of motor are provided in the motor box 205 and are configured to actuate the movement function of the robotic accessory 201. Regarding motors, each motor 219 for towing each wire 208, a motor for rotating the robotic accessory 201 about the longitudinal axis thereof which is not shown, and a motor for moving forward and backward the robotic accessory 201 in the longitudinal axial direction thereof which is not shown are used. Moreover, each encoder which is not shown is provided in each motor and is configured to measure a rotation angle of each motor. A pulley 224 is connected to the motor 219 for towing the wire and is configured to be rotated by the motor 219, and the wire 208 is wound around the pulley 224 so as to be towed by a rotation of the pulley 224. The wire 208 is coupled to the robotic accessory 201 through a wire coupling portion 223.

Each wire 208 is put out from the motor box 205 and put into a sheath portion 206 of the robotic accessory 201 through an outer connecting portion 204. The sheath portion 206 is long and flexible, and the distal end portion of the sheath portion 206 is coupled to the proximal end portion of an arm portion 207. In the arm portion 207 arms are coupled in order through joint portions 203 and each wire 208 is inserted through the sheath portion 206 and the arm portion 207 and connected to each joint portion 203. When the wires 208 are towed, the joint portions 203 are actuated to be rotated, the arms coupled to the joint portions 203 are driven, and a position and an attitude of the robotic accessory 201 are varied.

It is noted that a driving mechanism configured to drive the robotic accessory 201 may be any mechanism capable of driving the robotic accessory 201 such as a pneumatic actuator and an artificial muscle other than the wires 208 and the motor box 205.

Regarding a structure related to the treatment function of the robotic accessory 201, a high frequency electric knife 202 is used as an energy accessory and is configured to be controlled by a high frequency electric knife power apparatus 211.

That is, a counter electrode plate 216 and a foot switch 217 are connected to the high frequency electric knife power apparatus 211, and the counter electrode plate 216 is configured to be attached to the body surface of a patient to be operated and the foot switch 217 is configured to be pushed down by the foot of an operator to turned ON/OFF output of the high frequency electric knife 202. It is noted that a hand switch, a voice recognition switch and others may be used other than the foot switch 217 as an output instruction input means for operating output of the high frequency electric knife 202. The high frequency electric knife power apparatus 211 includes an output wattage input panel 214a configured to input output wattage to the high frequency electric knife 202, an output mode selection panel 214b configured to select an output mode of the high frequency electric knife 202 (an incision mode suitable for an incision dissect, a coagulation mode suitable for a hemostasis, and so on), and a display 213 configured to display information about a setting, output, and the like. An electric power output portion 215 of the high frequency electric knife power apparatus 211 is connected to the outer connecting portion 204 of the robotic accessory 201 through a high frequency electric knife code 21. When the electric power is supplied from the high frequency electric knife power apparatus 211 to the high frequency electric knife 202 provided on the distal end portion of the arm portion 207 of the robotic accessory 201, the high frequency electric knife 202 is actuated.

It is noted that a high frequency electric knife power apparatus communicating portion 218 of the high frequency electric knife power apparatus 211 is connected to a communicating portion for the high frequency electric knife power apparatus 241 of the robotic accessory control apparatus 220, and communication is performed between the high frequency electric knife power apparatus 211 and the robotic accessory control apparatus 220. ON/OFF of output, output wattage and an output mode of the high frequency electric knife power apparatus 211 are controlled on the basis of input to the foot switch 217, the output wattage input panel 214a and the output mode selection panel 214b in the normal mode, and further by the robotic accessory control apparatus 220 in the treatment function control mode.

Regarding a structure related to function control of the robotic accessory 201, the robotic accessory control apparatus 220 includes a function control input apparatus 233 configured to input the control parameters of the function control. That is, the function control input apparatus 233 includes a movement function control panel 230a for actuating/stopping movement function control, and a treatment function control panel 230b for actuating/stopping treatment function control. The function control input apparatus 233 further includes a reference point input timing panel 230c configured to instruct timing of a setting of the reference point as a reference in treatment by the robotic accessory 201, a reference direction input panel 230e configured to set a reference direction relative to the reference point, a reference distance input panel 230d configured to set the reference distance in the reference direction relative to the reference point, and the display 231 configured to display each setting.

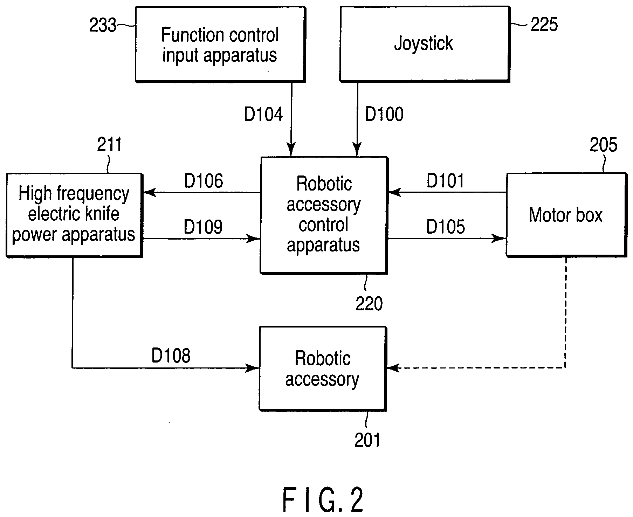

Referring to FIG. 2, control of the movement function and the treatment function of the robotic accessory 201 will be explained.

Regarding control of the movement function in the normal mode, the joystick 225 is manipulated by an operator, and therefore, target position data and target attitude data D100 of the distal end portion of the robotic accessory 201 are input to the robotic accessory control apparatus 220. On the other hand, rotation angle data D101 of each motor measured by each encoder is input from the motor box 205 to the robotic accessory control apparatus 220. The CPU 245 of the robotic accessory control apparatus 220 calculates position data and attitude data of the distal end portion of the robotic accessory 201 from the rotation angle data D101 of each motor, and compares the target position data and the target attitude data D100 of the robotic accessory 201 input from the joystick 225 and the actual position data and the actual attitude data. Next, a motor control signal D105 so as to reduce a difference between the target data and the actual data is input from the motor drive 247 of the robotic accessory control apparatus 220 to each motor of the motor box 205. Each motor is rotated, towing of wires 208, a rotation and a forward and a backward movement of the robotic accessory 201, and the like are performed, and therefore, the distal end portion of the robotic accessory 201 is moved to the target position and the target attitude input by the joystick 225. By repeating the above steps, a position and an attitude of the distal end portion of the robotic accessory 201 are made same as the target position and the target attitude.

It is noted that potentiometers and the like may be provided in the joint portions 203 of the robotic accessory 201 and a position and an attitude of the robotic accessory 201 may be detected directly using rotation angles of the joint portions 203 measured by the potentiometers and the like.

Regarding control of the treatment function in the normal mode, output wattage and an output mode are set by the output wattage input panel 214a and the output mode selection panel 214b of the high frequency electric knife power apparatus 211. The foot switch 217 is push down by an operator, and therefore, a high frequency current D108 having the set output wattage and the set output mode is output from the high frequency electric knife power apparatus 211 to the high frequency electric knife 202 and the high frequency electric knife 202 is actuated.

Regarding control of the movement function or the treatment function in the movement function control mode or the treatment function control mode, the robotic accessory control apparatus 220 is changed to the movement function control mode or the treatment function control mode by inputting to the movement function control panel 230a or the treatment function control panel 230b of the function control input apparatus 233.

Timing data, reference direction data and reference distance data D104 are input to the robotic accessory control apparatus 220 by inputting to the reference point input timing panel 230c, the reference direction input panel 230e and the reference distance input panel 230d of the function control input apparatus 233. The CPU 245 of the robotic accessory control apparatus 220 sets a position of the distal end of the robotic accessory 201 in the point of time when the timing data D104 is input as a reference point, and calculates a reference point data. As is mentioned above, the robotic accessory 201, the motor box 205, the function control input apparatus 233 and the robotic accessory control apparatus 220 forms a setting means for setting a reference point as a reference position and a reference direction.

The CPU 245 of the robotic accessory control apparatus 220 calculates distance data, velocity data, and acceleration data of the distal end portion of the robotic accessory 201 in the reference direction relative to the reference point on the basis of the rotation angle data D101 of each motor, the calculated reference point data and the calculated reference direction data D104. As is mentioned above, the robotic accessory 201, the motor box 205 and the robotic accessory control apparatus 220 form a detecting means for detecting a movement state of the robotic accessory 201 as a treatment accessory in the reference direction relative to the reference point as the reference position.

In the movement function control mode, as is mentioned in detail below, a motor control signal D105 to be input from the motor drive 247 of the robotic accessory control apparatus 220 to each motor of the motor box 205 is varied on the basis of the distance data, the velocity data or the acceleration data about the distal end of the robotic accessory 201 in the reference direction relative to the reference point.

Moreover, in the treatment function control mode, output ON/OFF data, output wattage data and output mode data D109 is output from the high frequency electric knife power apparatus 211 to the robotic accessory control apparatus 220. As is mentioned in detail below, an output control signal D106 for controlling the output ON/OFF, the output wattage and the output mode is output from the robotic accessory control apparatus 220 to the high frequency electric knife power apparatus 211 on the basis of the distance data, the velocity data or the acceleration data about the distal end of the robotic accessory 201 in the reference direction relative to the reference point. The output control signal D106 is prior to the input by the foot switch 217, the output wattage input panel 214a and the output mode selection panel 214b.

Next, a method for using the endoscopic surgical apparatus according to the embodiment will be explained.

Hereinafter, a case where the endoscopic surgical apparatus according to the embodiment is applied to an endoscopic submucosal dissection (shortly referred to as ESD, hereinafter) will be explained. ESD is a surgery wherein a lesion part in the stomach, the intestine, and so on is dissected as a whole under an endoscopic observation.

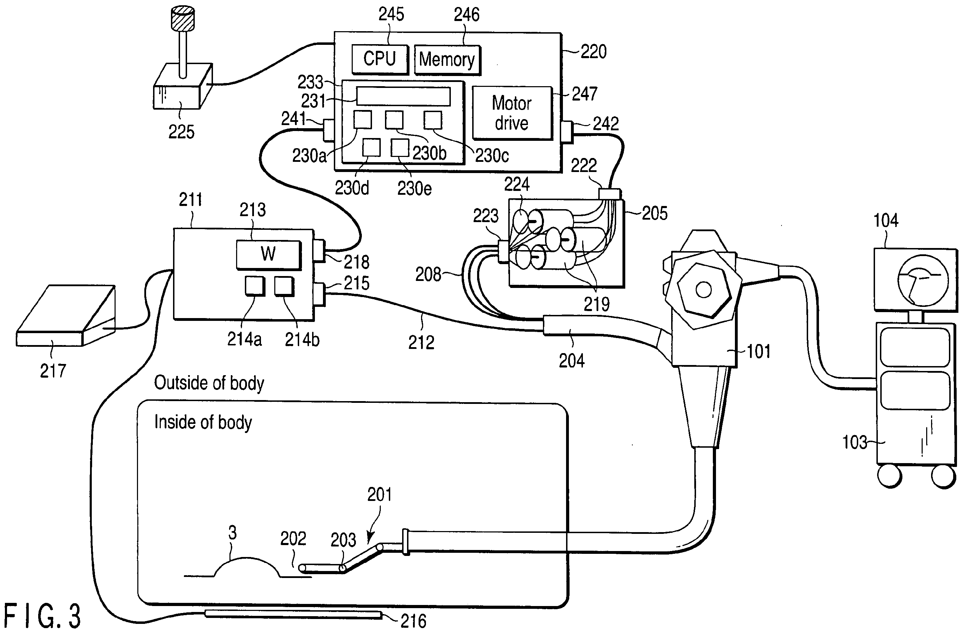

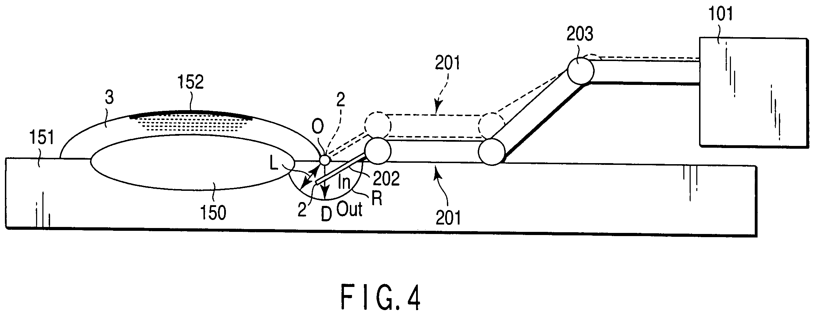

Referring to FIGS. 3 and 4, an endoscope 101 is connected to a video system center, a light source apparatus and the like carried by a trolley 103. Then, the endoscope 101 is perorally inserted into the inside of the stomach. Illumination light is supplied from the light source apparatus and emitted from the distal end portion of the endoscope 101, an observation image is picked-up by an image pick-up device in the distal end portion of the endoscope 101, an image signal is input to the video system center and then processed, and the observation image is displayed on a display apparatus 104. The distal end portion of the endoscope 101 is placed near a lesion part in the stomach, and then, various kinds of accessory are inserted into through a channel of the endoscope 101 and a physiological saline 150 is injected between a mucosa tissue 151 including a lesion part 152 and a muscular tunica propria to raise an affected part 3.

Next, the robotic accessory 201 is inserted into the channel of the endoscope 101, and the arm portion 207 of the robotic accessory 201 is projected from the distal end portion of the endoscope 101. The sheath portion 206 of the robotic accessory 201 is housed in the channel. Then, the endoscope 101 is operated to be placed on a position wherein the robotic accessory 201 can reach the affected part 3.

In the normal mode, an operator manipulates the joystick 225 while observing the observation image on the display apparatus 104, and therefore, the robotic accessory 201 is moved following the manipulation to the joystick 225. Moreover, the operator pushes down the foot switch 217, and the high frequency electric knife 202 is actuated with output wattage and an output mode set in the high frequency electric knife power apparatus 211.

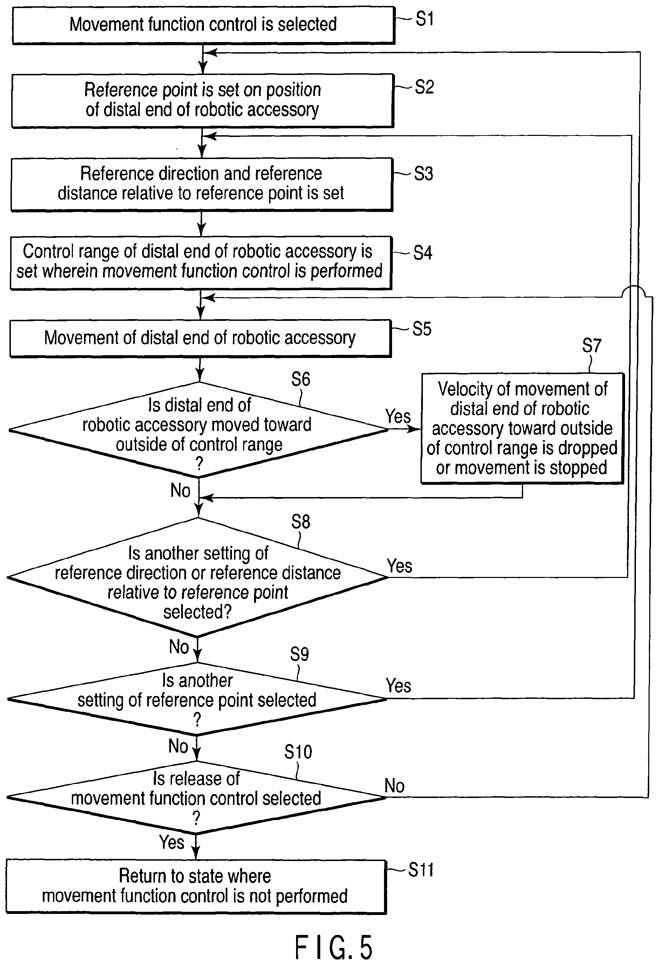

Referring to FIG. 5, control of the movement function of the movement function control mode will be explained.

Step 1 (S1)

The movement function control panel 230a of the function control input apparatus 233 is manipulated and the movement function control is selected, and therefore, the robotic accessory control apparatus 220 is changed from the normal mode to the movement function control mode.

Step 2 (S2)

The joystick 225 is manipulated to move the distal end 2 of the robotic accessory 201 to a position which is to be set as a reference point. In the embodiment, as is shown by a dotted line in FIG. 4, in order to set a position where the distal end 2 of the robotic accessory 201 is brought into contact with the surface of a mucosa tissue 151 of a plane part near a part from which raising is started of the affected part 3 as a reference point O, it is moved to the above position. In this state, the reference point input timing panel 230c is manipulated to set and storage the position of the reference point O. The reference point O is used as long as a reference point O is set again.

Step 3 (S3)

A reference direction and a reference distance are set through the reference direction input panel 230e and the reference distance input panel 230d of the function control input apparatus 233. In the embodiment, as is shown in FIG. 4, a direction which is an outwardly radial direction about the reference point O and faces a body wall side is selected as a reference direction D, a movement distance which is permissible when the distal end of the robotic accessory 201 is moved in the reference direction D from the reference point O is selected as a reference distance L. The above reference direction D or the above reference distance L is used as long as a reference direction D or a reference distance L is set again.
A reference direction and a reference distance may be preset.

Step 4 (S4)

A region wherein distance in the reference direction from the reference point is the reference distance L and below is set as a control range wherein the movement function control of the distal end of the robotic accessory 201 is performed. A region wherein distance in the reference direction from the reference point is the reference distance and below is the inside of the control range and a region wherein it is over the reference distance is the outside of the reference range. Therefore, the reference direction is a direction toward the outside of the control range. In the embodiment, as is shown in FIG. 4, a hemispherical range having radius L about the reference point O and arranged on the body wall side is set as a control range R, the inside of the above hemispherical range is the inside of the control range R, the outside thereof is the outside of the control range R.

Step 5 (S5)

As is shown by a solid line in FIG. 4, the joystick 225 is manipulated to move the distal end 2 of the robotic accessory 201.

Step 6 (S6)

The robotic accessory control apparatus 220 detects whether the distal end of the robotic accessory 201 is moved toward the outside of the control range R or not.

Step 7 (S7)

When the distal end of the robotic accessory 201 is moved toward the outside of the control range R, in addition to manipulation by the joystick 225, as is mentioned in detail below, control is performed wherein velocity of a movement of the distal end of the robotic accessory 201 toward the outside of the control range R is made to be dropped or the movement is made to be stopped.

Step 8 (S8)

When another setting of a reference direction or a reference distance is selected, the step returns to Step 3 (S3) and the movement function control is performed on the basis of the reference direction and the reference distance set again.

Step 9 (S9)

When another setting of a reference point is selected, the step returns to Step 2 (S2) and the movement function control is performed on the basis of the reference point O set again.

Steps 10 and 11 (S10 and S11)

When release of the movement function control is not selected, the step returns to Step 5 (S5), and the similar movement function control is continued.
When release of the movement function control is selected, the state returns to the state where the movement function control is not performed.

Next, control will be explained in detail wherein velocity of the movement of the distal end of the robotic accessory 201 is made to be dropped or the movement is made to be stopped when the distal end of the robotic accessory 201 is moved toward the outside of the control range R.

Here, distance and velocity of the distal end of the robotic accessory 201 in the reference direction relative to the reference point are referred as Ls and Vs.

Control will be explained wherein velocity Vs is made to be dropped according to an increase in distance Ls.

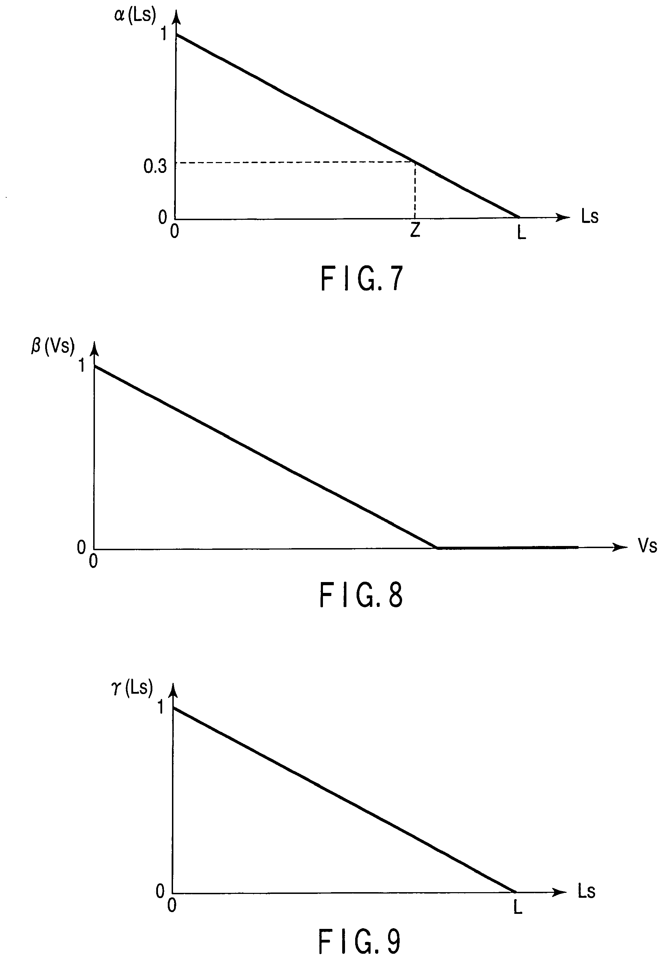

As is shown in the formula (1), new velocity Vsnew is calculated by multiplying velocity Vs by a variable α(Ls) (0≤α(Ls)≤1) which decreases according to an increase in distance Ls. An example of the variable α(Ls) is shown in FIG. 7. The robotic accessory 201 is controlled such that the velocity of the distal end of the robotic accessory 201 is made to be new velocity Vsnew.

$$\text{Vsnew} = \alpha(\text{Ls}) \times \text{Vs} \qquad (1)$$

For example, when the distance Ls from the reference point O is Z and the value of α(Ls) is 0.3, those are substituted into the formula (1), and as a result,

$$\text{Vsnew} = 0.3 \times \text{Vs} \qquad (2)$$

wherein the new velocity is 30 percent of the original velocity.

In the above control, although the new velocity Vsnew is calculated by multiplying the original velocity Vs by the variable α(Ls) which is 0 or over and 1 or less, various variables wherein the new velocity Vsnew is made to be smaller than the original velocity Vs according to an increase of the distance Ls may be used.

As is shown in the formula (3), the new velocity Vsnew may be calculated by subtracting a variable K(Ls) (0≤K(Ls)≤|Vs|) which increases according to an increase in the distance Ls from the original velocity Vs. Here, the original velocity Vs and the new velocity Vsnew are equal with each other in the sign.

$$\text{Vsnew} = \frac{\text{Vs}}{|\text{Vs}|}(|\text{Vs}| - \text{K(Ls)}) \qquad (3)$$

Control will be explained wherein velocity Vs is dropped according to an increase of the velocity Vs.

As is shown in the formula (4), new velocity Vsnew is calculated by multiplying the original velocity Vs by a variable β(Vs) (0≤β(Vs)≤1) which decreases according to an increase in the original velocity Vs. An example of the variable β(Vs) is shown in FIG. 8. The robotic accessory 201 is controlled such that the velocity of the distal end of the robotic accessory 201 is made to be the new velocity Vsnew.

$$\text{Vsnew} = \beta(\text{Vs}) \times \text{Vs} \qquad (4)$$

In the above control, although the new velocity Vsnew is calculated by multiplying the original velocity Vs by the variable β(Vs) which is 0 or over and 1 or below, various variables may be used wherein the new velocity Vsnew is made to be smaller than the original velocity Vs according to an increase in the original velocity Vs. For example, the new velocity Vsnew may be calculated by subtracting a variable which increases according to an increase in the original velocity Vs from the original velocity Vs. However, the original velocity Vs and the new velocity Vsnew are equal with each other in the sign.

In the above control, the original velocity Vs is directly converted to the new velocity Vsnew. Furthermore, regarding the distal end of the robotic accessory 201, original distance or original acceleration is converted to new distance or new acceleration according to a similar corresponding relationship to that in the case of the velocity on the basis of distance or acceleration in the reference direction relative to the reference point. The acceleration may be made to be decreased according to an increase in the acceleration in the reference direction relative to the reference point. The various kinds of control of the movement function mentioned above are used in combination.

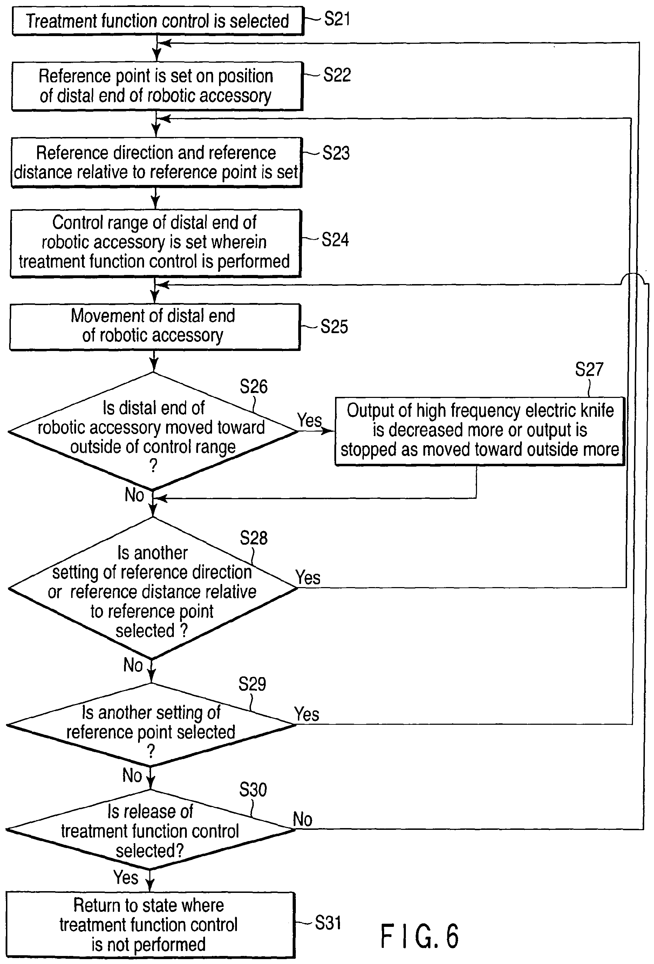

Referring to FIG. 6, control of the treatment function of the treatment function control mode will be explained.

Step 21 (S21)

The treatment function control panel 230b of the function control input apparatus 233 is manipulated to select the treatment function control and the robotic accessory control apparatus 220 is changed from the normal mode to the treatment function control mode.

Steps 22 to 26 (S22 to S26)

As is similar to the case of the movement function control, a reference point, a reference direction and a reference distance is set, and a control range is set wherein the treatment function control of the distal end of the robotic accessory 201 is performed. When the robotic accessory 201 is moved, whether the distal end of the robotic accessory 201 is moved toward the outside of the control range or not is detected.

Step 27 (S27)

When the distal end of the robotic accessory 201 is moved toward the outside of the control range, with priority to input by the foot switch 217 and the output wattage input panel 214a, as is mentioned in detail below, output wattage of the high frequency electric knife power apparatus 211 to the high frequency electric knife 202 is made to be decreased or output is made to be stopped, and output of the high frequency electric knife 202 is made to be decreased or stopped.

Steps 28 and 29 (S28 and S29)

As is similar to the case of the movement function control, when another setting of the reference direction or the reference distance and the reference point is selected, the step returns to Step 23 (S23) or Step 22 (S22), and the control of the treatment function is performed on the basis of the reference direction, the reference distance and the reference point set again.

Steps 30 and 31 (S30 and S31)

When release of the treatment function control is not selected, the step returns to Step 25 (S25) and the similar treatment function control is continued.

When release of the treatment function control is selected, the state of the robotic accessory 201 returns to the state where the treatment function control is not performed.

Next, control will be explained in detail wherein the output wattage of the high frequency electric knife power apparatus 211 is made to be decreased or the output is made to be stopped, and therefore, the output of the high frequency electric knife 202 is made to be decreased or stopped when the distal end of the robotic accessory 201 is moved toward the outside of the control range R.

Regarding a decrease in the output wattage, the output wattage may be decreased using an analog system, or ON/OFF of the high frequency electric knife power apparatus 211 may be switched at sufficiently high speed in comparison with the velocity of the movement of the distal end of the robotic accessory 201 and the average output wattage may be decreased by changing the ratio of the ON-time to the OFF-time.

Here, the output wattage of the high frequency electric knife power apparatus 211 is referred as W, and the acceleration of the distal end of the robotic accessory 201 in the reference direction D relative to the reference point O is referred as As.

Control will be explained wherein the output wattage W of the high frequency electric knife power apparatus 211 is made to be decreased according to an increase in the distance Ls from the reference point O to the distal end of the robotic accessory 201 in the reference direction D.

As is shown in the formula (5), new output wattage Wnew is calculated by multiplying original output wattage W set in the high frequency electric knife power apparatus 211 by a variable $\gamma(Ls)$ ($0 \leq \gamma(Ls) \leq 1$) which decreases according to an increase in the distance Ls. The output from the high frequency electric knife power apparatus 211 to the high frequency electric knife 202 is performed at the new output wattage Wnew. FIG. 9 shows an example of the variable $\gamma(Ls)$.

$$\text{Wnew} = \gamma(\text{Ls}) \times \text{W} \qquad (5)$$

In the above control, although the new output wattage Wnew is calculated by multiplying the original output wattage W by the variable $\gamma(Ls)$, various functions may be used wherein the new output wattage Wnew is made to be smaller than the original output wattage W according to an increase in the distance Ls.

As is shown in the formula (6), the new output wattage Wnew is calculated by subtracting a variable J(Ls) ($0 \leq$ J(Ls)$\leq$W) which increases according to an increase in the distance Ls from the original output wattage W. However, the new output wattage Wnew is made to be 0 or over.

$$Wnew = W - J(Ls) \qquad (6)$$

Control will be explained wherein the output wattage W of the high frequency electric knife power apparatus 211 is made to be decreased according to an increase in the velocity Vs of the distal end of the robotic accessory 201 in the reference direction D relative to the reference point O.

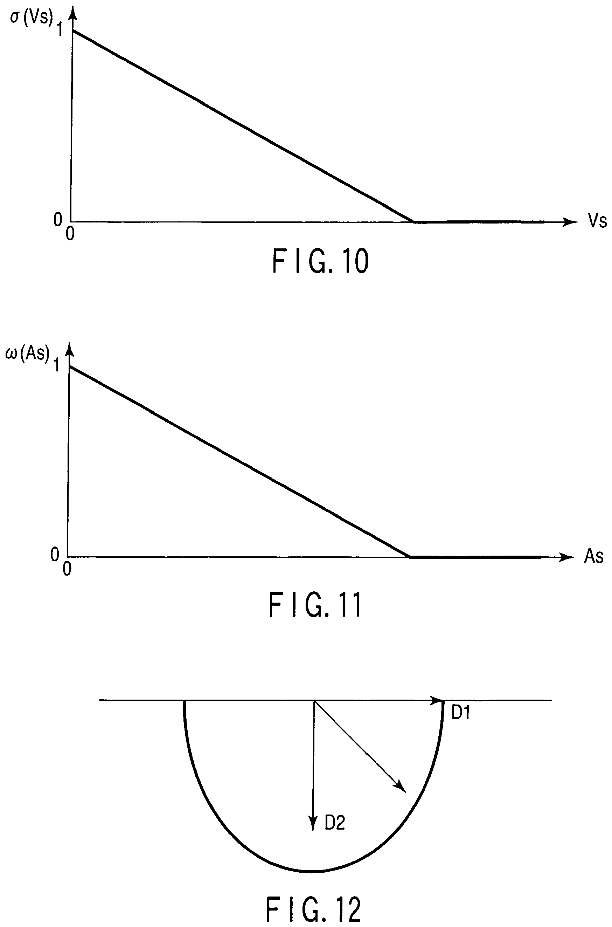

As is shown in the formula (7), the new output wattage Wnew is calculated by multiplying the original output wattage W set in the high frequency electric knife power apparatus 211 by a variable o(Vs) ($0 \leq$ σ(Vs)$\leq$1) which decreases according to an increase of the velocity Vs. The output from the high frequency electric knife power apparatus 211 to the high frequency electric knife 202 is performed at the new output wattage Wnew. FIG. 10 shows an example of the variable o(Vs).

$$Wnew = \sigma(Vs) \times W \qquad (7)$$

In the above control, although the new output wattage Wnew is calculated by multiplying the original output wattage W by the variable o(Vs), various variables may be used wherein the new output wattage Wnew is made to be smaller than the original output wattage W according to an increase in the velocity Vs. The new output wattage Wnew may be calculated by subtracting a variable which increases according to an increase in the velocity Vs from the original output wattage W. However, the new output wattage Wnew is made to be 0 and over.

Control will be explained wherein the output wattage W of the high frequency electric knife power apparatus 211 is made to be decreased according to an increase in acceleration As of the distal end of the robotic accessory 201 in the reference direction D relative to the reference point O.

As is shown in the formula (8), the new output wattage Wnew is calculated by multiplying the original output wattage W set in the high frequency electric knife power apparatus 211 by a variable ω(As) ($0 \leq$ω(As)$\leq$1) which decreases according to an increase in the acceleration As. The output from the high frequency electric knife power apparatus 211 to the high frequency electric knife 202 is performed at the new output wattage Wnew. FIG. 11 shows an example of the variable ω(As).

$$Wnew = \omega(As) \times W \qquad (8)$$

In the above control, the new output wattage Wnew is calculated by multiplying the original output wattage W by the variable ω(Vs), various variables may be used wherein the new output wattage Wnew is made to be smaller than the original output wattage W according to an increase in the acceleration As. The new output wattage Wnew may be calculated by subtracting a variable which increases according to an increase in the acceleration As from the original output wattage W. However, the new output wattage Wnew is made to be 0 and over.

In the above mentioned control, although the output wattage of the high frequency electric knife power apparatus 211 is varied, the output mode is varied with priority to the output mode set in the high frequency electric knife power apparatus 211. That is, in the high frequency electric knife 202, incision frequency suitable for an incision dissection and coagulation frequency suitable for a hemostasis are combined at a certain ratio to be used, and, in the high frequency electric knife power apparatus 211, the ratio of the incision frequency may be made to be decreased to drop an incision capability according to an increase in distance, velocity, acceleration of the distal end of the robotic accessory 201 in the reference direction D relative to the reference point O.

Therefore, the endoscopic surgical apparatus according to the embodiment exhibits the following effect.

In the endoscopic surgical apparatus according to the embodiment, the movement function control or the treatment function control is used, and therefore, it is possible to prevent an movement of the robotic accessory 201 and an excessive incision by the high frequency electric knife 202 due to carelessness and a mistake of an operator, whereby enabling to perform accuracy treatment. Moreover, it is possible to raise treatment speed and lighten a mental burden of an operator since the operator can perform treatment with a sense of safety.

Moreover, the reference point can be set on a position where the distal end of the robotic accessory 201 is placed, and therefore, it is possible to recognize the position of the surface of the mucosa tissue 151 by setting the reference point in the state where the distal end of the robotic accessory 201 is in contact with the surface of the mucosa tissue 151.

It is noted that the movement function control is what controls a movement function of an accessory, and therefore, the movement function control is not limited to an energy accessory whose distal end includes a high frequency electric knife, a high frequency snare and others, and it is applicable to a robotic accessory whose distal end includes a knife-shaped or a needle-shaped scalpel, forceps and so on. On the other hand, the treatment function control is what controls the treatment function of the accessory, and therefore, the treatment function control is not limited to the robotic accessory, and it is applicable to various energy accessories wherein the distal end thereof is movable and the movement state is detectable.

Moreover, regarding the timing of the setting of the reference point, other than an manipulation to the reference point input timing panel 230c, a pressure sensor may be provided on the distal end of the robotic accessory 201, an instant when the distal end of the robotic accessory 201 is brought into contact with the surface and the like of the mucosa tissue 151 may be detected and the reference point may be set on the position of the distal end of the robotic accessory 201 at this instant.

FIGS. 12 to 16 show a second embodiment of the present invention.

In the first embodiment, one reference direction is used and, for example, the linear variables as is shown in FIGS. 7 and 9 are used as $\alpha(Ls)$ in the formula (1) and $\gamma(Ls)$ in the formula (5). However, it is not necessary that the variable is linear, and also, a plurality of directions is used as the reference direction and variables different from each other are used for the reference directions, respectively. For example, it is most important in treatment such as ESD to prevent boring to a tunica muscularis propria, and it is necessary to enhance an effect of a function control, in particular, with respect to a depth direction of a mucosa tissue 151.

In the embodiment, as is shown in FIG. 12, a horizontal direction along the surface of the mucosa tissue 151 of a plane part near a part from which raising is started of an affected part 3 is set as a first reference direction D1 or 0 degree direction, and a depth direction perpendicular to the surface of the mucosa tissue 151 is set as a second reference direction D2 or 90 degree direction. Distances and velocities of the distal end 2 of a robotic accessory 201 in the 0 degree direction and the 90 degree direction from a reference point are referred as L1s, L2s, V1s and V2s.

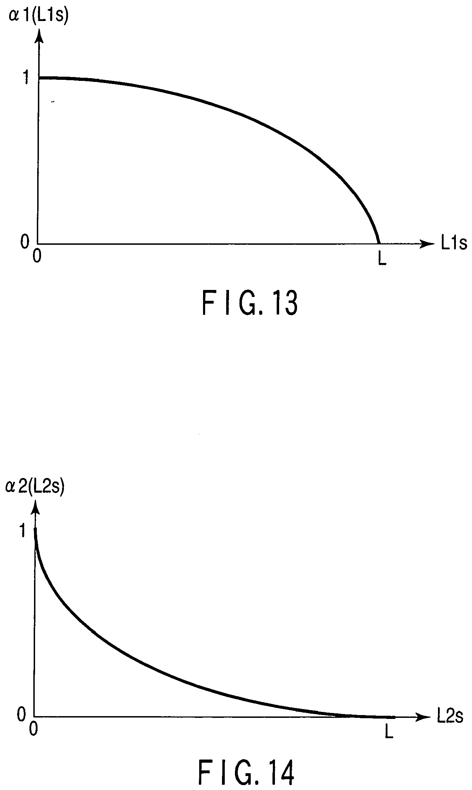

Referring to the formula (1), regarding the 0 degree direction, V1snew = $\alpha$1(L1s) $\times$ V1s, and, regarding the 90 degree direction, V2snew = $\alpha$2(L2s) $\times$ V2s. Regarding a movement of the distal end of the robotic accessory 201 in the 0 degree direction, as is shown in FIG. 13, $\alpha$1(L1s) forms a gentle decreasing curve up to the neighborhood of the outside of a reference distance L, that is, a control range R. On the other hand, regarding a movement of the distal end of the robotic accessory 201 in the 90 degree direction, as is shown in FIG. 14, $\alpha$2(L2s) forms a decreasing curve which decreases suddenly even a little apart from the reference point O. By using such variable $\alpha$1(L1s) and $\alpha$2(L1s), a movement function control property is realized wherein it is easy to move in the 0 degree direction and it is hard to move in the 90 degree direction. Regarding P(Vs) as is shown in the formula (4), variables different form each other may be used for the 0 degree direction and the 90 degree direction as is similar to $\alpha$(Ls), and therefore, a similar effect may be obtained.

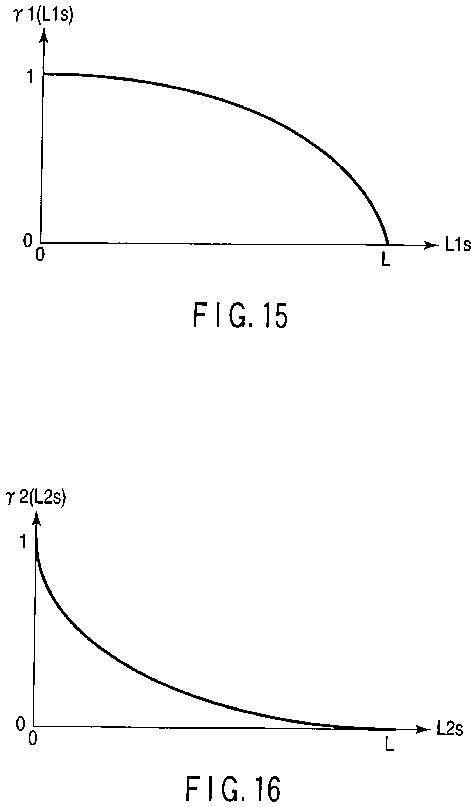

Moreover, referring to the formula (5), Wnew = $\gamma$1(L1s) $\times$ $\gamma$2(L2s) $\times$ W. Regarding a movement of the distal end of the robotic accessory 201 in the 0 degree direction, as is shown in FIG. 15, $\gamma$1(L1s) forms a gentle decreasing curve up to the neighborhood of the outside of the reference distance L, that is, the control range R. On the other hand, regarding a movement of the distal end of the robotic accessory 201 in the 90 degree direction, as is shown in FIG. 16, $\gamma$2(L2s) forms a decreasing curve which decreases suddenly even a little apart from the reference point O. By using such variable $\gamma$1(L1s), $\gamma$2(L1s), a treatment function control property is realized wherein it is easy to incise in the 0 degree direction and it is hard to incise in the 90 degree direction. Regarding o(Vs) and $\omega$(As) as is shown in the formulas (7) and (8), variables different from each other may be used for the 0 degree direction and the 90 degree direction as is similar to $\gamma$(Ls), and therefore, a similar effect may be obtained.

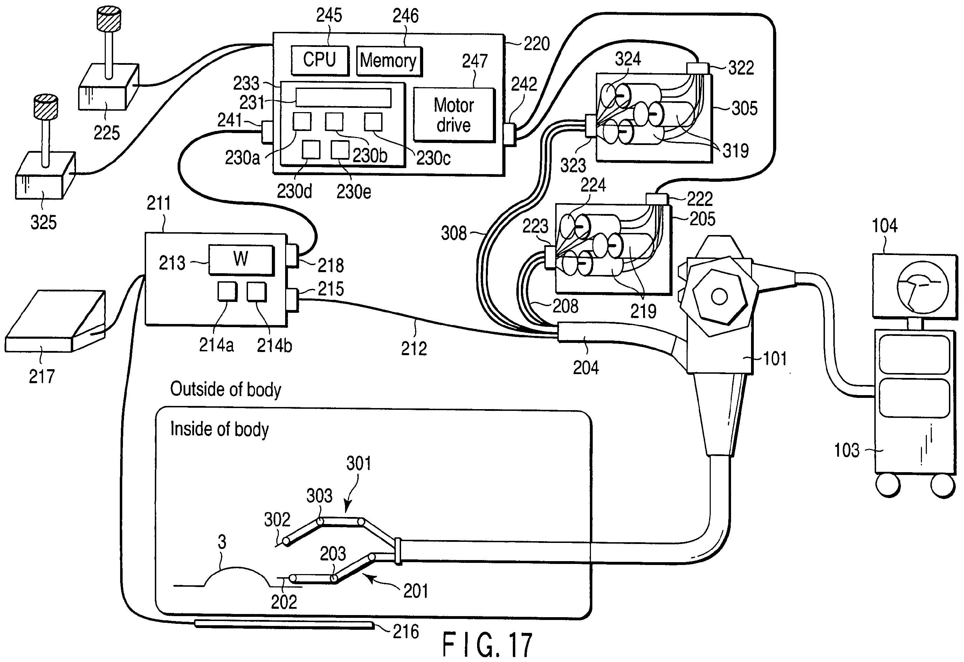

FIGS. 17 to 20 show a third embodiment of the present invention.

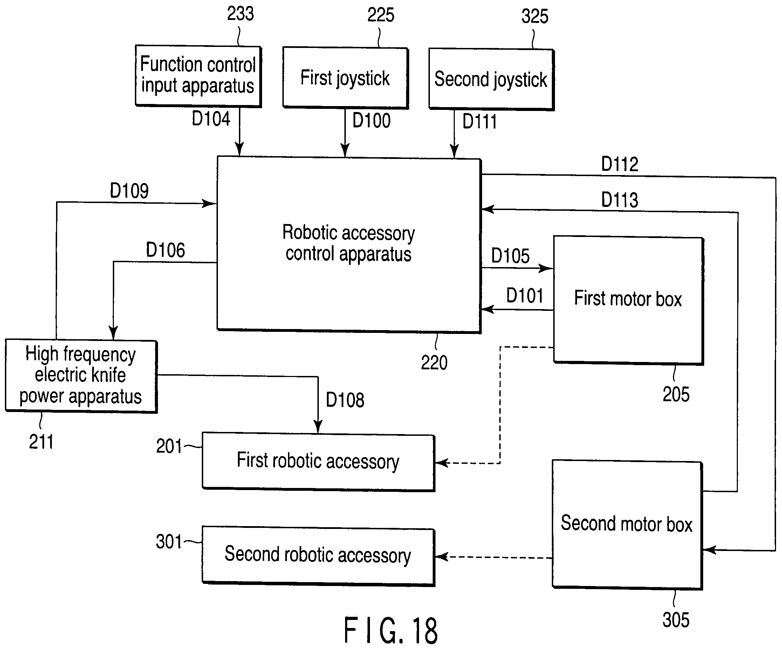

Referring to FIGS. 17 and 18, an endoscopic surgical apparatus according to the embodiment includes a first robotic accessory 201, a first motor box 205 and a first joystick 225 similar to those according to the first embodiment, and further, a second robotic accessory 301, a second motor box 305 and a second joystick 325 having structures similar to those of those. However, a high frequency electric knife is not provided on the distal end portion of the second robotic accessory 301. The first robotic accessory 201 is used as a treatment accessory for performing treatment and the second robotic accessory 301 is used as a setting accessory for setting a reference point.

Here, regarding the second robotic accessory 301, the second motor box 305 and the second joystick 325, components having similar functions to those of components 2xx of the first robotic accessory 201, the first motor box 205 and the first joystick 225 are denoted by references 3xx and explanation will be omitted.

As is similar to the first robotic accessory 201, a target position data and a target attitude data D111 of the distal end of the second robotic accessory 301 are input from the second joystick 325 to a robotic accessory control apparatus 220. On the other hand, a rotation angle data D113 of each motor measured by each encoder is input from the second motor box 305 to the robotic accessory control apparatus 220. A motor control signal D112 is input from a motor drive 247 of the robotic accessory control apparatus 220 to each motor of the second motor box 305.

In the embodiment, CPU 245 of the robotic accessory control apparatus 220 sets a position where the distal end 12 of the second robotic accessory 301 is placed when a timing data D104 is input through a reference point input timing panel 230c as a reference point, and calculates a reference point data. That is, in the embodiment, the second robotic accessory 301, the second motor box 305, the function control input apparatus 233 and the robotic accessory control apparatus 220 forms a setting means.

Next, a method for using an endoscopic surgical apparatus according to the embodiment is explained taking ESD as an example.

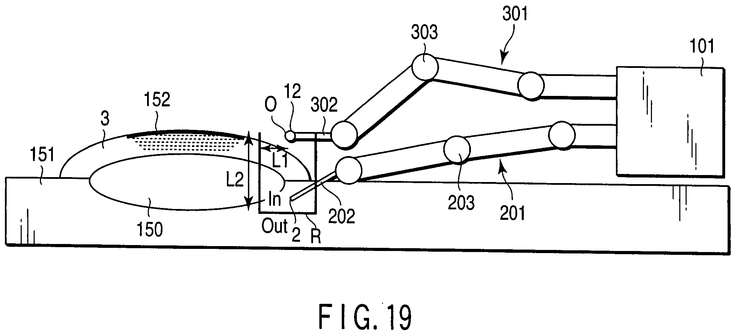

Referring to FIG. 19, as is similar to the first embodiment, an endoscope 101 is inserted into to the position which is suitable for observing an affected part 3. The first and the second robotic accessory 201 and 301 is inserted into a first and a second channel of the endoscope 101, respectively, and is projected from the distal end portion of the endoscope 101.

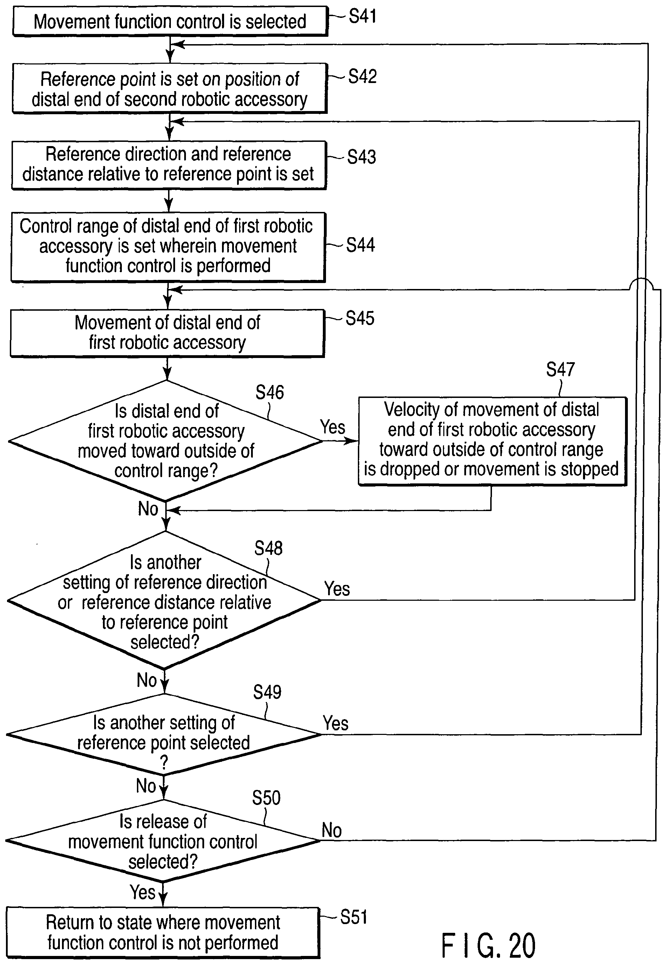

Referring to FIG. 20, control of a movement function in a movement function control mode will be explained.

Step 41 (S41)

As is similar to the first embodiment, the movement function control is selected.

Step 42 (S42)

The second joystick 325 is manipulated to move the distal end 12 of the second robotic accessory 301 to a position to be set as a reference point. In the embodiment, as is shown in FIG. 19, in order to set a reference point O on a position near a part from which raising is started of an affected part 3, the distal end 12 of the second robotic accessory 301 is moved to the above position. In this state, the reference point input timing panel 230c is manipulated to set and storage a position of a reference point. The reference point is used as long as a reference point is set again.

Step 43 (S43)

A reference direction and a reference distance is set through a reference direction input panel 230e and a reference distance input panel 230d of a function control input apparatus 233. In the embodiment, as is shown in FIG. 19, a direction parallel with the surface of a mucosa tissue 151 of a plane part near a part from which raising is started is set as a first reference direction and a direction perpendicular to the surface of the mucosa tissue 151 and toward a body wall side is set as a second reference direction. Regarding the first and the second reference direction, movement distances wherein a movement of the distal end of the robotic accessory 201 is permissible are a first and a second reference distance L1 and L2, respectively. The reference directions or the reference distances are used as long as reference directions or reference distances are set again.

Reference directions and reference distances may be preset.

Step 44 (S44)

As is similar to the first embodiment, a control range of the distal end 2 of the first robotic accessory 201 wherein the movement function control is performed, and the inside and the outside of the control range are set on the basis of the reference point, the reference directions and the reference distances. As is shown in FIG. 19, the control range R according to the embodiment forms a circularly columnar shape whose central axis is an axis perpendicular to the surface of the mucosa tissue 151 and passing through the reference point O, whose radius is L1 and which extends over the distance L2 from the reference point O in a direction perpendicular to the surface of the mucosa tissue 151 toward the body wall side and the inside and the outside of the circularly columnar shape forms the inside and the outside of the control range R, respectively.

Steps 45 to 48 (S45 to S48)

As is similar to the first embodiment, the movement function of the first robotic accessory 201 is controlled on the basis of the set control range.

Moreover, when another setting of the reference direction or the reference distance is selected, the step returns to Step 43 (S43).

Step 49 (S49)

When another setting of the reference point is selected, the step returns to Step 42 (S42), and the second joystick 325 is manipulated again to move the distal end 12 of the second robotic accessory 301 to a position to be set as a reference point and the reference point input timing panel 230c is manipulated to perform a setting. Then, the movement function control is performed on the basis of the reference point set again.

Steps 50 and 51 (S50 and S51)

As is similar to the first embodiment, when release of the movement function control is not selected, the step returns to Step 45 (S45), the similar movement function control is continued, and, when release of the movement function control is selected, the state returns to the state where the movement function control is performed.

In the embodiment, a treatment function control similar to that according to the second embodiment is performable. That is, as the distal end 2 of the first robotic accessory 201 is moved toward the outside of the control range more, output of a high frequency electric knife 202 of the first robotic accessory 201 is made to be decreased more or stopped, or, the ratio of coagulation frequency to incision frequency is varied to drop an incision capability. However, as is similar to the case of the above mentioned movement function control, a reference point is set by the distal end 12 of the second robotic accessory 301.

Therefore, the endoscopic surgical apparatus according to the embodiment exhibits the following effect in addition to the effects of the first embodiment.

In the embodiment, treatment is performed by the first robotic accessory 201 and the reference point and the control range in treatment is set by the second robotic accessory 301, and therefore, it is possible to frequently change the reference point and the control range in treatment while performing treatment. Therefore, it is possible to improve reliability and increase speed of treatment, and further decrease a mental burden of an operator.

In the embodiment, although the reference point is set at the timing input through the reference point input timing panel 230c, a reference point may be set continuously and automatically on a position where the distal end 12 of the second robotic accessory 301 is placed. In this case, the reference point can be set automatically just by moving the distal end 12 of the second robotic accessory 301 without manipulating the reference point input timing panel 230c, and therefore, it is possible to further easily and frequently perform a change in the reference point and the control range.

Moreover, although the second robotic accessory 301 is used only for the setting of the reference point, the second robotic accessory 301 may be used as a grasping accessory or an energy accessory, for example, a grasping forceps, or a high frequency electric knife or an ultrasonic scalpel may be provided on the distal end of the second robotic accessory 301.

Furthermore, the reference point may be set by the distal end 2 of the first robotic accessory 201 in addition to the distal end 12 of the second robotic accessory 301. In the case where the second robotic accessory 301 is used as a treatment accessory, a reference point and a control range set by the distal end 2 of the first robotic accessory 201 may be a reference point and a control range of the second robotic accessory 301, and the reference point and the control range set by the distal end 12 of the second robotic accessory 301 may be a reference point and a control range of the first and the second robotic accessory 201 and 301. Moreover, a common reference point and a common control range may be used regarding the first or the second robotic accessory 201 or 301.

Furthermore, three or more above robotic accessory, for example, a third and a fourth robotic accessory having similar structures to those of the first and the second robotic accessory may be used. It is possible to properly select which of the robotic accessories is used and which of reference points and control ranges for the robotic accessories is set by each of selected accessories.

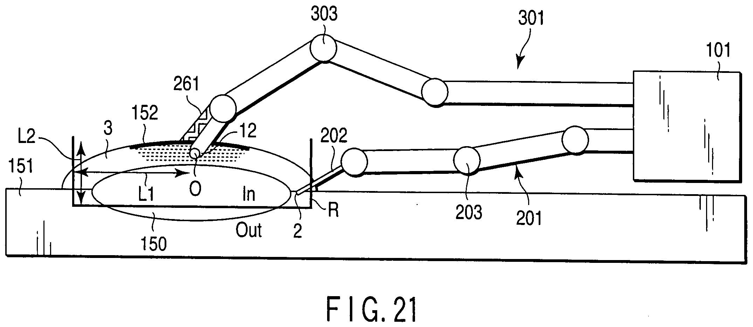

FIG. 21 shows a modified example of the third embodiment of the present invention.

A grasping forceps 261 is provided on the distal end of a second robotic accessory 301 according to the modified example. The middle part of a raising part of an affected part 3 is grasped by the grasping forceps 261 and, in this state, a reference point input timing panel 230c is manipulated to set a reference point O, and therefore, the reference point O is set on the position grasped by the grasping forceps 261. Then, a first and a second reference direction is set as is similar to the third embodiment, and a reference distance L1 is set at a length which is as long as a radius of the raising part with respect to the first reference direction parallel with the surface of a mucosa tissue 151. A control range is set as is mentioned above, and therefore, it is possible for the first robotic accessory 201 to perform a circular incision dissection.

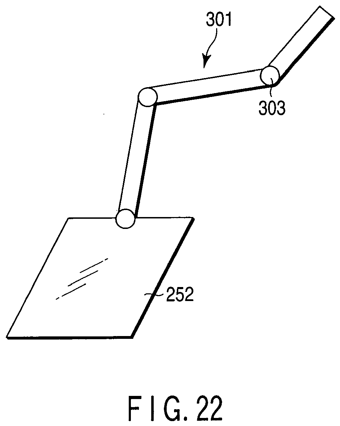

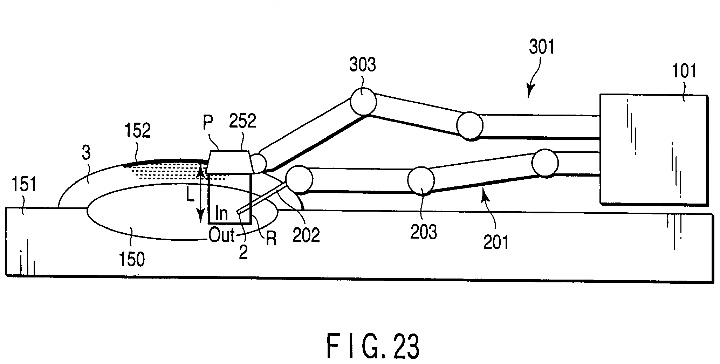

FIGS. 22 and 23 show a fourth embodiment not according to the present invention.

Referring to FIG. 22, a flat board-shaped setting portion 252 is provided on the distal end of a second robotic accessory 301 according to the embodiment. A reference plane as a reference position is set on a position where one plane of the setting portion 252 is placed. Referring to FIG. 23, in ESD, the setting portion 252 is pushed onto a raising affected part 3 and placed parallel to the surface of a mucosa tissue 151 of a plane part near a part from which raising is started. Then, a reference plane P is set on a position where the one plane of the setting portion 252 is placed, a direction perpendicular to the surface of the mucosa tissue 151 and toward the body wall side is set as a reference direction, and therefore, a rectangular parallelepiped-shape control range R is set. Therefore, it is possible to easily perform a setting of a control range in a depth direction from a raising part of an affected part 3.

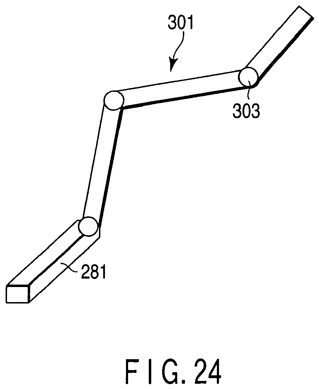

FIG. 24 shows a modified example of the fourth embodiment not according to the present invention.

Referring to FIG. 24, a rod-shaped setting portion 281 is provided on the distal end portion of a second robotic accessory 301 according to the modified example, and it is possible for the rod-shaped setting portion 281 to set a reference line as a reference position.

As is mentioned above, a line-shaped reference line, a flat board-shaped reference plane as well as a point-shaped reference point may be used as a reference position. Other than setting of the reference line or the reference plane by the above rod-shaped or the above flat board-shaped setting portion 281 or 252, a reference line may be set by setting two reference points, and a reference plane may be set by setting three reference points.

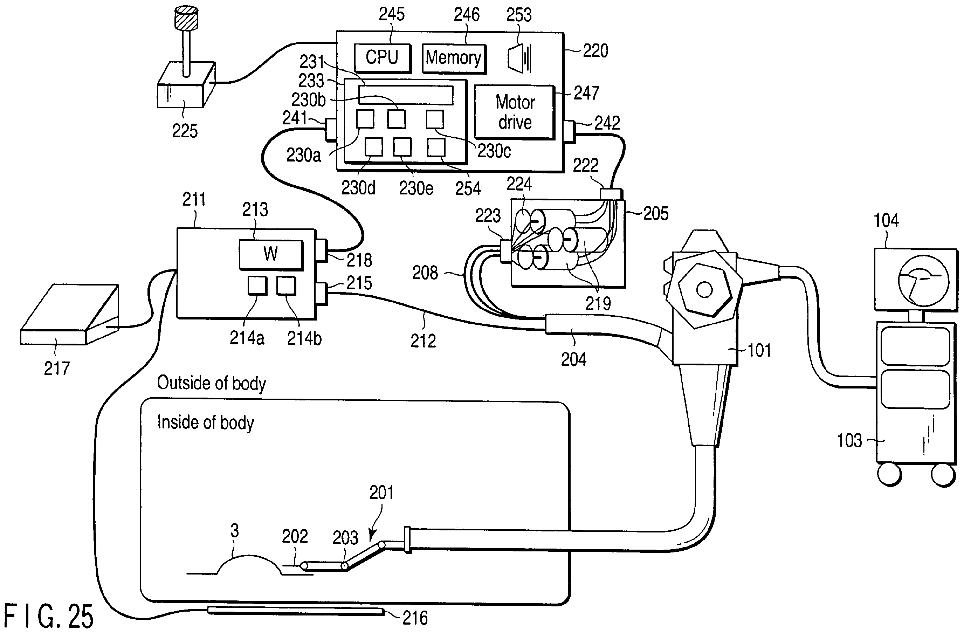

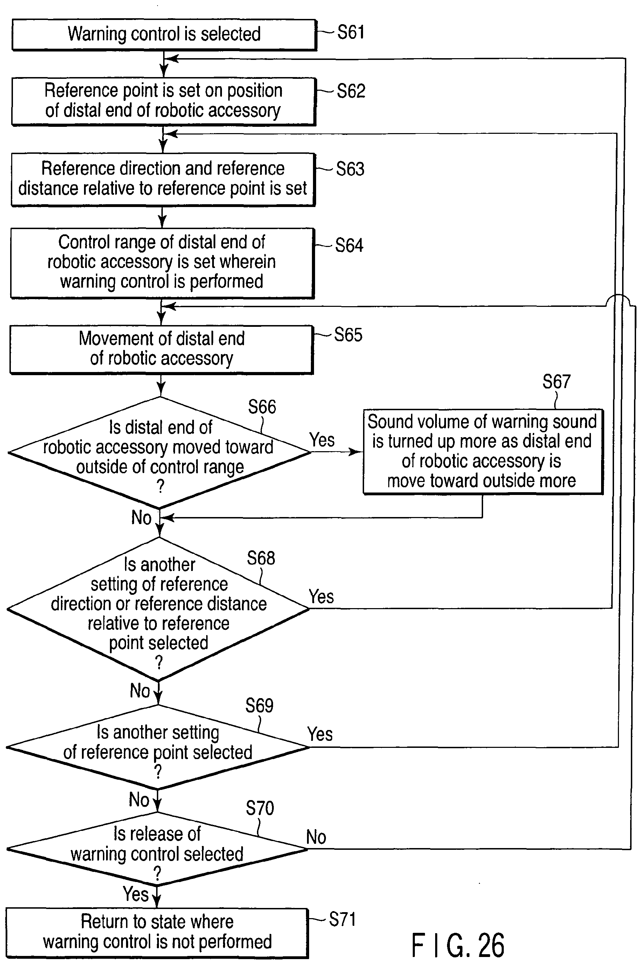

FIGS. 25 and 26 show a fifth embodiment not according to the present invention.

Referring to FIG. 25, an endoscope system according to the embodiment has a structure similar to that of the endoscope system according to the first embodiment, and in addition, a speaker 253 as a warning means is provided in a robotic accessory control apparatus 220 and configured to generate a warning. Furthermore, a warning control panel 254 is provided on a function control input apparatus 233 and configured to actuate/stop warning control.

Referring to FIG. 26, the warning control in a warning control mode will be explained.

Step 61 (S61)

The warning control panel 254 of the function control input apparatus 233 is manipulated to select the warning control, and therefore, the robotic accessory control apparatus 220 is changed from a normal mode to the warning control mode.

Steps 62 to 66 (S62 to S66)

As is similar to the first embodiment, a reference point, a reference direction and a reference distance is set, and a control range is set.

Whether the distal end of a robotic accessory 201 is moved toward the outside of the control range or not is detected.

Step 67 (S67)

When the distal end of the robotic accessory 201 is moved toward the outside of the control range, a warning sound is emitted from the speaker 253. As the distal end of the robotic accessory 201 is moved toward the outside of the control range more, sound volume of the warning sound is turn up more.

Steps 68 and 69 (S68 and S69)

As is similar to the first embodiment, when another setting of the reference direction, or the reference distance or the reference point is selected, the step returns to Step 63 (S63) or Step 62 (S62), and the warning control is performed on the basis of the reference direction, the reference distance and the reference point set again.

Steps 70 and 71 (S70 and S71)

When release of the warning control is not selected, the step returns to Step 65 (S65), and the similar warning control is continued.

When release of the warning control is selected, the state returns to the state where the warning control is not performed.

Therefore, the endoscopic surgical apparatus according to the embodiment exhibits the following effects.

In the embodiment, the warning control is used, and therefore, as is similar to the first embodiment, it is possible to prevent a movement of the robotic accessory 201 and an excessive incision by a high frequency electric knife 202 due to carelessness or mistake of an operator, whereby enabling to perform accuracy treatment. Moreover, it is possible to raise speed of treatment and lighten a mental burden of an operator since the operator can perform treatment with a sense of safety.

Moreover, when the robotic accessory 201 is moved toward the outside of the control range, a movement function of the robotic accessory 201 is not directly controlled and the warning sound is emitted to only excite attention regarding a movement, and therefore, a movement of the robotic accessory 201 is not hindered, and it is possible for a skilled operator to raise speed of treatment sufficiently.

It is noted that the warning control is not limited to an energy accessory and applicable to a robotic accessory whose distal end includes a knife shaped and a needle shaped scalpel, and forceps, and also, not limited to the robotic accessory and applicable to various accessories whose distal end is movable and whose movement state is detectable.

Although the sound volume of the warning sound is varied in the embodiment, a music interval, a combination of sound volume and a music interval, a melody and the like may be varied. Moreover, although the speaker is used as the warning means, light may emitted by a light and others and brightness, a color and so on of the light may be varied, vibrations may be generated by a vibration mechanism and amplitude and frequency of the vibrations may be varied, and a warning message may be displayed on a display. Furthermore, the robotic accessory control apparatus 220 may be connected to a display apparatus 104 configured to display an observation image of an endoscope to enable to perform communication with each other, and a warning which is variable according to a movement state of the distal end of the robotic accessory 201 may be displayed on the display apparatus 104.

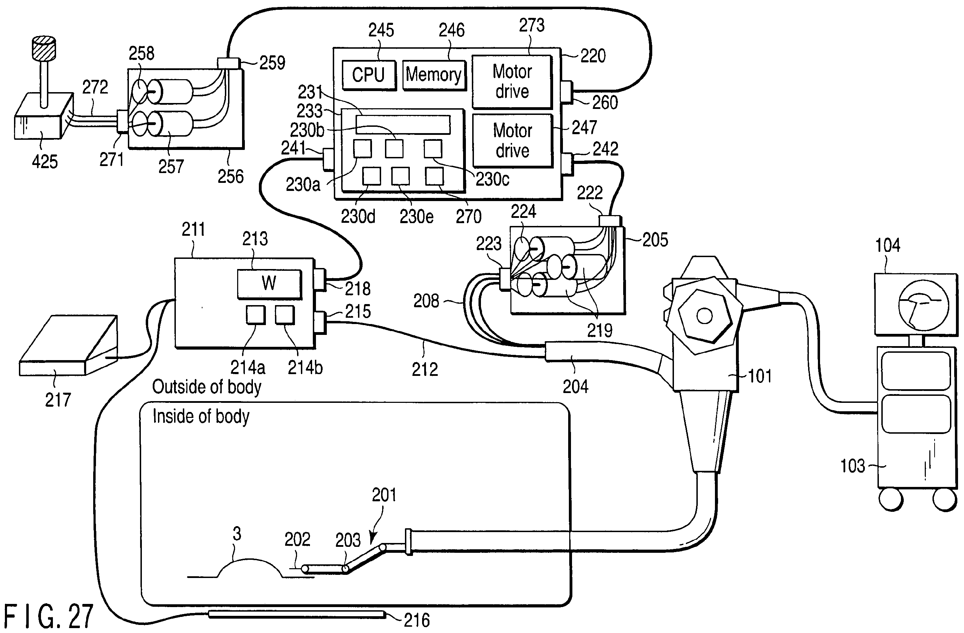

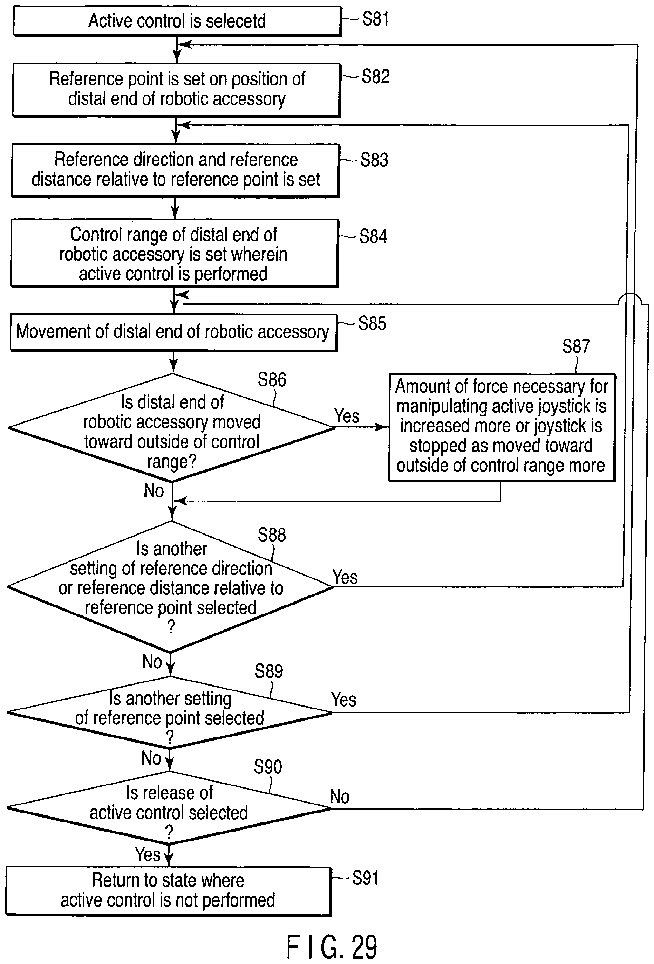

FIGS. 27 to 29 show a sixth embodiment not according to the present invention.

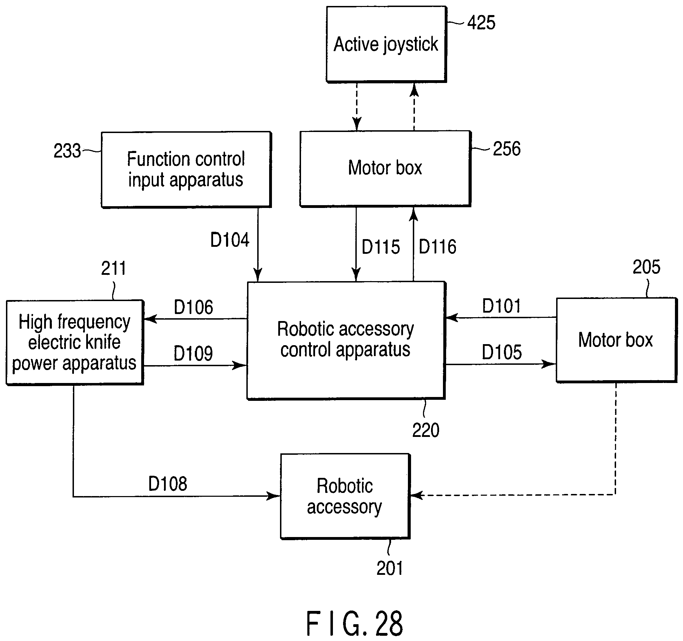

Referring to FIGS. 27 and 28, in an endoscopic surgical apparatus according to the embodiment, an active joystick 425 as an operating means is used. That is, wires 272 are put out from the active joystick 425, put into a motor box 256, and wound around pulleys 258 of motors 257, respectively. When the active joystick 425 is manipulated, each wire 272 is towed and each motor 257 is rotated through each pulley 258. On the other hand, when each motor 257 rotates each pulley 258 to tow each wire 272, reaction force against manipulation to the active joystick 425 is varied or a manipulatable range of the active joystick 425 is limited. Each encoder which is not shown is provide in each motor 257 and configured to measure a rotation angle of each motor 257. A motor box communicating portion 259 of the motor box 256 is connected to a communicating portion for a motor drive 260 of a robotic accessory control apparatus 220, and communication is performable between the motor box 256 and the robotic accessory control apparatus 220. The robotic accessory control apparatus 220 includes a motor drive 273 configured to control the motors 257 of the motor box 256. Moreover, an active control panel 270 is provided on a function control input apparatus 233 and configured to actuate/stop active control.

Referring to FIG. 28, control of the active joystick 425 will be explained.

In a normal mode, the active joystick 425 is manipulated, each wire 272 is towed, and each motor 257 is rotated through each pulley 258. Rotation angle data D115 measured by each encoder on each motor 257 is input from the motor box 256 to the robotic accessory control apparatus 220. CPU 245 of the robotic accessory control apparatus 220 calculates target position data and target attitude data of the distal end portion of the robotic accessory 201 from the rotation angle data D115. After that, as is similar to the first embodiment, the robotic accessory 201 is moved to the target position and the target attitude.

By input to the active control panel 270 of the function control input apparatus 233, the robotic accessory control apparatus 220 is changed to an active control mode. In the active control mode, as is similar to the first embodiment, the CPU 245 of the robotic accessory control apparatus 220 calculates distance data and velocity data of the distal end of the robotic accessory 201 in a reference direction relative to a reference point. Motor control signal D116 is input from the motor drive 273 of the robotic accessory control apparatus 220 to the motor box 256 on the basis of the calculate distance data and the calculate velocity data. As is mentioned in detail below, each motor 257 is controlled on the basis of the motor control signal D116 and reaction force against manipulation to the active joystick 425 is varied or a manipulatable range of the active joystick 425 is limited through the pulley 258 and the wire 272.

Next, a method for using the endoscopic surgical apparatus according to the embodiment is explained.

Step 81 (S81)

The active control panel 270 of the function control input apparatus 233 is manipulated to select the active control and change the robotic accessory control apparatus 220 from the normal mode to the active control mode.

Steps 82 to 86 (S82 to S86)

As is similar to the first embodiment, a reference point, a reference direction and a reference distance is set, and a control range is set.

Whether the distal end of the robotic accessory 201 is moved toward the outside of the control range or not is detected.

Step 87 (S87)

When the distal end of the robotic accessory 201 is moved toward the outside of the control range, in order to make manipulation of the active joystick 425 hard or unable, amount of manipulation force necessary for manipulation to the active joystick 425 is made to be increased or the active joystick 425 is forced to be stopped.

Steps 88 and 89 (S88 and S89)

As is similar to the first embodiment, when another setting of the reference direction or the reference distance, or the reference point is selected, the step returns to Step 83 (S83) or Step 82 (S82), and the active control is performed on the basis of the reference direction and the reference distance, and the reference point set again.

Steps 90 and 91 (S90 and S91)

When release of the active control is not selected, the step returns to Step 85 (S85), and the similar active control is continued.

When release of the active control is selected, the state returns to the state where the active control is not performed.

Therefore, the endoscopic surgical apparatus according to the embodiment exhibits the following effect.

In the embodiment, the active control is used, and therefore, as is similar to the first embodiment, it is possible to prevent a movement of the robotic accessory 201 and an excessive incision by a high frequency electric knife 202 due to carelessness or a mistake of an operator, whereby enabling to perform accuracy treatment. Moreover, it is possible to raise speed of treatment and lighten a mental burden of an operator since the operator can perform treatment with a sense of safety.

It is noted that the active control is not limited to an energy accessory and applicable to a robotic accessory whose distal end includes a knife shaped and a needle shaped scalpel, forceps and so on.

A modified example of the sixth embodiment not according to the present invention will be explained.

In the modified example, a motor is provided in a foot switch 217 and is configured to be rotated according to pushing down of the foot switch 217. In a treatment function control mode, when the distal end of a robotic accessory 201 is moved toward the outside of a control range, a robotic accessory control apparatus 220 controls the motor to make amount of operation force necessary for pushing down of the foot switch 217 increased or to force pushing down stopped in order to make pushing down of the foot switch 217 hard or unable.

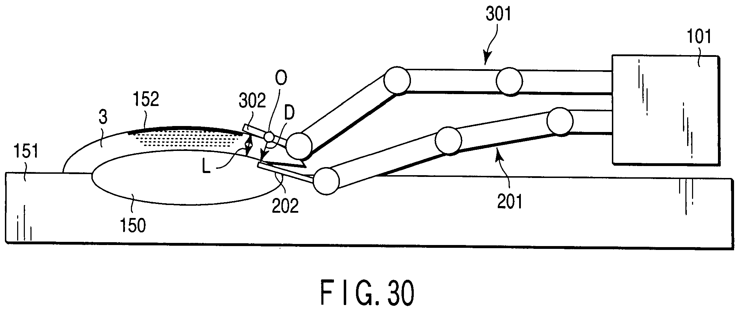

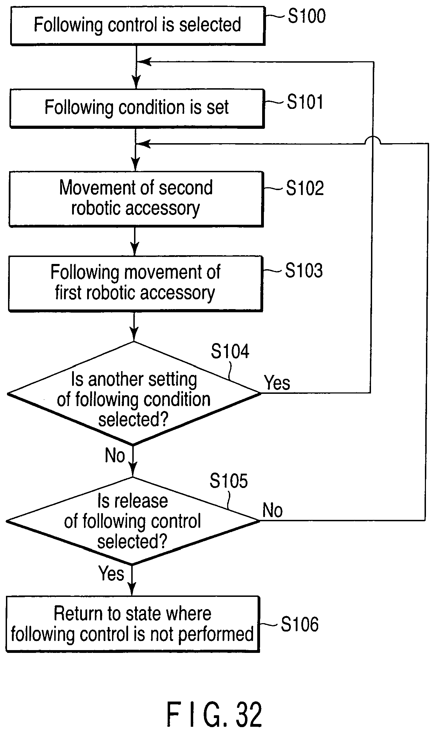

FIGS. 30 to 32 show a seventh embodiment not according to the present invention.

An endoscopic surgical apparatus according to the embodiment includes a similar structure to that of the endoscopic surgical apparatus according to the third embodiment shown in FIGS. 17 and 18, and it is possible to automatically move a first robotic accessory 201 following a second robotic accessory 301. That is, a following control panel configured to actuate/stop following control and a following condition input panel configured to set a following condition are provided on a function control input apparatus 233. In the embodiment, a position and an attitude of a second robotic accessory 301 is set as a reference position and a reference attitude for treatment, a direction, a distance and an attitude of the first robotic accessory 201 relative to the second robotic accessory 301 is used as the following condition. A robotic accessory control apparatus 220 is configured to calculate position data and attitude data of the second robotic accessory 301 as is similar to the third embodiment in a following control mode and to output a motor control signal to a first motor box 205 to drive the first robotic accessory 201 on the basis of the calculated position data and the calculated attitude data, and the set following condition.

Next, a method for using the endoscopic surgical apparatus according to the embodiment will be explained taking ESD as an example.

Step 100 (S100)

The following control panel of the function control input apparatus 233 is manipulated to select the following control, and the robotic accessory control apparatus 220 is changed from a normal mode to the following control mode.

In the embodiment, as is shown in FIG. 30, a setting portion 302 of the second robotic accessory 301 is pushed onto the surface of the peripheral part of a raising part of an affected part 3 and an incision from the peripheral part of the raising part of the affected part 3 to the inside thereof is started by a high frequency electric knife 202 of the first robotic accessory 201, and, at this state, a change from the normal mode to the following control mode is performed.

Step 101 (S101)

The following condition input panel of the function control input apparatus 233 is manipulated to set a direction, a distance and an attitude of the first robotic accessory 201 relative to the second robotic accessory 301 as a following condition. In the embodiment, as is shown in FIG. 30, a direction toward the inside of the affected part 3 is set as a following direction D and a thickness of a mucosa tissue 151 is set as a following distance L with respect to the setting portion 302 of the second robotic accessory 301 pushed onto the surface of the peripheral part of the raising part of the affected part 3, and, regarding a following attitude, an attitude of the high frequency electric knife 202 of the first robotic accessory 201 is made to be similar to that of the setting portion 302 of the second robotic accessory 301.

It is noted that a relative position of the first robotic accessory 201 relative to the second robotic accessory 301 at the time of the start of the following control may be used as a following direction and a following distance, and also, an attitude of the first robotic accessory 201 at the time of the start of the following control may be used as it is as a following attitude, or a following attitude may not be set in the case where it is not necessary to give consideration to an attitude. Moreover, a following condition may be preset.

Step 102 (S102)

The second joystick 325 is manipulated to move the second robotic accessory 301. In the embodiment, the setting portion 302 of the second robotic accessory 301 is moved along the surface of the raising part of the affected part 3.

Step 103 (S103)

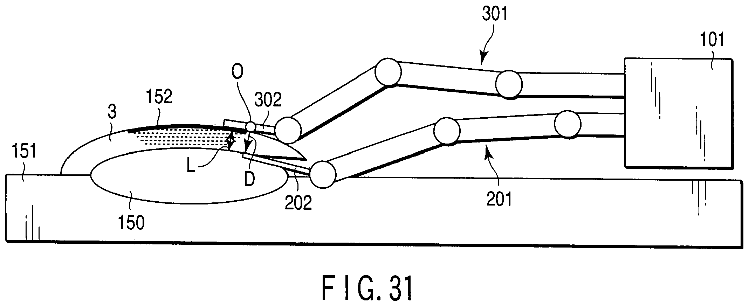

The first robotic accessory 201 is moved according to a movement of the second robotic accessory 301 and the set following condition. In the embodiment, as is shown in FIG. 31, the high frequency electric knife 202 of the first robotic accessory 201 follows the setting portion 302 of the second robotic accessory 301 in the state where the former is apart from the latter by the thickness of the lesion part and the former has the same attitude as the latter. As a result, the inside of the affected part 3 is incised by the high frequency electric knife 202 and the only mucosa tissue 151 is incised to be dissected.

Step 104 (S104)

When another setting of the following condition is selected, the step returns to Step 101 (S101), and the following control is performed on the basis of the following condition set again.

Steps 105 and 106 (S105 and S106)

When release of the following control is selected, the step returns to Step 102 (S102), and the similar following control is performed.
When release of the following control is selected, the state returns to the state where a following control is not performed.
Therefore, the endoscopic surgical apparatus according to the embodiment exhibits the following effect.
In the endoscopic surgical apparatus according to the embodiment, the following control is used, and therefore, it is possible to operate the high frequency electric knife 202 of the first robotic accessory 201 in the state it is not observable by an endoscope 101 by operating the setting portion 302 of the second robotic accessory 301 in the state where it is observable by the endoscope 101.
In particular, in the embodiment, the direction toward the inside of the affected part 3 is set as the following direction, the thickness of the mucosa tissue 151 is set as the following distance, the setting portion 302 of the second robotic accessory 301 is pushed onto the raising part of the affected part 3 and moved along it, and the mucosa tissue 151 is incised by the high frequency electric knife 202 of the first robotic accessory 201, and therefore, it is possible to incise the only mucosa tissue 151 including the lesion part. Therefore, the high frequency electric knife 202 is prevented from being moved toward a tunica muscularis propria due to carelessness or a mistake of an operator, and it is possible to perform an incision while reducing amount of raising of the affected part 3.
It is noted that it is not necessary that the first robotic accessory 201 is made to follow the second robotic accessory 301 at the same velocity and the former may be made to follow the latter slowly. In the case of the slow following, it is possible for an operator to recognize surely situation of an incision.
Moreover, a plane board-shaped setting portion 252 as is shown in FIG. 22 may be used as the setting portion 302 of the second robotic accessory 301. Such plane board-shaped setting portion 252 is easy to be placed parallel to a shape of a raising part of an affected part 3 when it is pushed onto the affected part 3, and it is possible to recognize a direction toward the inside of the affected part 3.

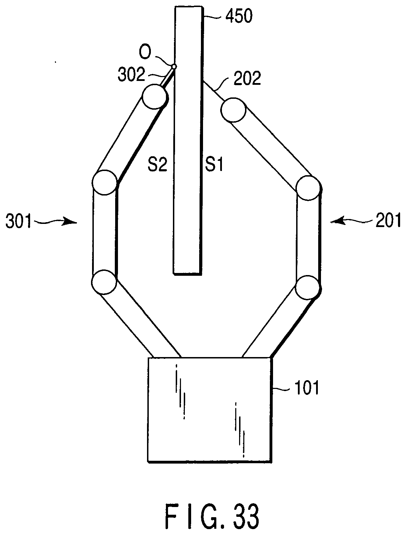

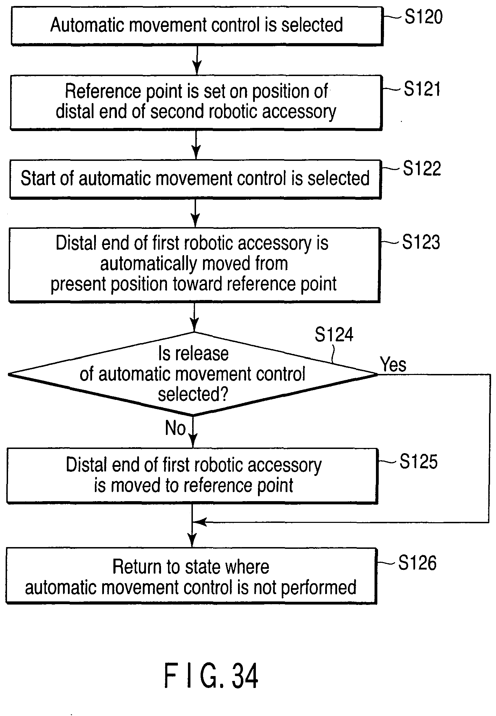

FIGS. 33 and 34 show an eighth embodiment not according to the present invention.
An endoscopic surgical apparatus according to the embodiment has a structure similar to that of the endoscopic surgical apparatus according to the third embodiment shown in FIGS. 17 and 18, and in addition, it is possible to automatically move the distal end of a first robotic accessory 201 toward a reference point.

That is, a function control input apparatus 233 includes an automatic movement control panel configured to actuate/stop an automatic movement control and an automatic movement starting panel configured to actuate an automatic movement of the first robotic accessory 201. A robotic accessory control apparatus 220 is configured to move the distal end of the first robotic accessory 201 toward a set reference point with priority to manipulation to a first joystick 225 in an automatic movement control mode. It is noted that, although a preset condition is used as a movement condition such as velocity and acceleration in the automatic movement of the distal end of the first robotic accessory 201, a movement condition input panel may be provided on the function control input apparatus 233 and a movement condition may be appropriately set.

Next, a method for using the endoscopic surgical apparatus according to the embodiment will be explained taking an operation wherein a lumen wall is penetrated by an accessory as an example.

Referring to FIG. 33, the distal end of the first robotic accessory 201 is moved to an automatic movement starting position. In the embodiment, the first robotic accessory 201 is inserted into a lumen, and the distal end of the first robotic accessory 201 is moved to a penetration starting position on a lumen side S1.

Step 120 (S120)

The automatic movement control panel is manipulated to select the automatic movement control, and the robotic accessory control apparatus 220 is changed from a normal mode to the automatic movement control mode.

Step 121 (S121)

The second joystick 325 is manipulated to move the distal end of the second robotic accessory 301 to a position to be set as a reference point. The reference point is an automatic move ending position of the distal end of the first robotic accessory 201. In the embodiment, as is shown in FIG. 33, the distal end of the second robotic accessory 301 is moved to a penetration ending position of the distal end of the first robotic accessory 201 in an abdominal cavity side S2. In this state, a reference point input timing panel 230c is manipulated to set and storage the position of the reference point. The reference point is used as long as a reference point is set again.

After setting the reference point, if necessary, the second robotic accessory 301 is retracted from the reference point.

Step 122 (S122)

The automatic movement starting panel is manipulated to start an automatic movement of the distal end of the first robotic accessory 201.

Step 123 (S123)

The distal end of the first robotic accessory 201 is automatically moved toward the reference point. In the embodiment, a high frequency electric knife 202 of the first robotic accessory 201 is moved while incising a lumen wall 450 from the penetration starting position to the penetration ending position.

Step 124 (S124)

When the automatic movement control panel is manipulated to select release of the automatic movement control in the middle of the movement of the distal end of the first robotic accessory 201, the movement of the distal end of the first robotic accessory 201 is stopped, the step advances to Step 126 (S126), and the state returns to the state where the automatic movement control is not performed.

Step 125 (S125)

When the distal end of the first robotic accessory 201 is moved to the reference point, the automatic movement is stopped. In the embodiment, a penetrating bore is formed through the lumen wall 450.

Step 126 (S126)

The state returns to the state where the automatic movement control is not performed.

Therefore, the endoscopic surgical apparatus according to the embodiment exhibits the following effect.

In the endoscopic surgical apparatus according to the embodiment, the automatic movement control is used, and therefore, it is possible for an accessory to penetrate the lumen wall 450 giving consideration to states in both planes on the abdominal cavity side and the lumen side of the lumen wall 450. For example, in the case where there is a blood vessel on the abdominal cavity side in the lumen wall 450, it is possible for the accessory to penetrate the lumen wall 450 while avoiding the blood vessel by setting the reference point on a position sufficiently apart form the blood vessel. Moreover, the distal end of the first robotic accessory 201 can be moved accurately to the aimed penetration ending position even when both the sides of the lumen wall 450 can not be observed simultaneously and the penetration ending position can not be observed, and therefore, it is possible to improve a safety in penetrating by the accessory. Furthermore, before an approach of a treatment portion with an incision function to a mucosa tissue 151 and so on, the reference point can be set by an approach of a treatment portion with no incision function to the mucosa tissue 151 and so on, and therefore, it is possible to prevent the treatment portion with the incision function from incising an unnecessary part.

It is noted that a movement of the distal end of the robotic accessory toward the reference point is applicable to various usages such as an incision and a dissection to a mucosa tissue 151 other than the penetration through the lumen wall 450.

Regarding each of the above mentioned embodiments, various modifications may be embodied as follows.

Regarding setting of a reference position, various particular positions in a robotic accessory may be used such as a position of a joint of the robotic accessory and a position of a marker arranged on the robotic accessory other than a position of the distal end of the robotic accessory. An exclusive accessory for setting of a reference position may be used. Moreover, a virtual robotic accessory and a virtual laser marker displayed on a display may be used. Furthermore, a positional information generating apparatus configured to generate three-dimensionally positional information from an observation image of an endoscope may be carried on a trolley, a position may be designated on the observation image on a display apparatus, and therefore, the reference position may be set on the basis of the generated three-dimensional information. A three-dimensional position and a three-dimensional attitude of a robotic accessory may be calculated by such positional information generating apparatus.

Although control is performed on the basis of a position of the distal end of a robotic accessory relative to a control range in any of a movement function control, a treatment function control, a warning control, an active control and an automatic movement control, control may be performed on the basis of various particular positions of the robotic accessory such as a position of a joint of the robotic accessory and a position of a marker arranged on the robotic accessory other than the distal end of the robotic accessory.

**Claims**

1. An endoscopic surgical apparatus comprising:

   a treatment accessory (201; 301) configured to be inserted through and project from a channel of an endoscope (101), including a treatment portion with a treatment function, and having a movement function;
   a setting means (201, 205, 220, 233) configured to set: either

   - a reference position (O) as a reference in treatment by the treatment accessory (201) and a reference direction (D) as a direction from the reference position (O) into a mucosa tissue (151) and orthogonal to the surface of the mucosa tissue (151), and a reference distance (L) relative to the reference position (O), and configured to set a region as a hemispherical control range (R) with points having a distance from the reference position (O) which is the reference distance (L) or below, wherein the surface of the mucosa tissue (151) represents the flat side of the hemisphere; or
   - a reference position (O) as a reference in treatment by the treatment accessory (301), a first and second reference directions, the first reference direction being a direction parallel to the surface of the mucosa tissue (151) of a plane part near a part from which raising of an affected part (3) is started and the second reference direction being a direction perpendicular to the surface of the mucosa tissue (151) and toward a body wall side, and first and second reference distances (L1, L2) relative to the reference position (O) and a reference axis passing through the reference position (O), and configured to set a region as a columnar control range (R) which extends over the second reference distance (L2) from the reference position (O) with points having a distance from the reference axis which is the first reference distance (L1) or below;

   a detecting means (201, 205, 220) configured to detect whether the treatment accessory (201; 301) is moved toward the outside of the control range (R) or not; and
   a control means (220) configured to control a movement of the treatment accessory (201; 301) toward the outside of the control range (R) in a reference direction, which is made to be decelerated or stopped, or a treatment capability of the treatment portion, which is made to be decreased or stopped, when the treatment accessory (201; 301) is moved toward the outside of the control range (R) in the reference direction, wherein the reference direction is:

the reference direction (D) from the reference position (O) into a mucosa tissue (151) and orthogonal to the surface of the mucosa tissue (151), when the control range (R) is the hemispherical control range, or the direction of distance (L2) away from the reference point (O), respectively the direction of distance (L1) away from the reference axis, when the control range (R) is the columnar control range.

**2.** The endoscopic surgical apparatus according to claim 1, wherein the setting means (201, 205, 220, 233; 301, 305, 220, 233) is configured to set a plurality of reference directions (D1, D2), and a variable of deceleration or stop of the movement of the treatment accessory (201; 301) or a variable of decrement or stop of the treatment capability with respect to the movement for the plurality of reference directions (D1, D2) are different from each other.

**3.** The endoscopic surgical apparatus according to claim 1, wherein the setting means (201, 205, 220, 233; 301, 305, 220, 233) includes a setting accessory (301) with a setting portion (252; 281; 302) for setting the reference position (O) and is configured to set the reference position (O) on a position where the setting portion (252; 281; 302) is placed.

**4.** The endoscopic surgical apparatus according to claim 1, wherein the treatment accessory (201; 301) includes an energy accessory (202) and the treatment capability includes magnitude of output or a mode of output.

**5.** The endoscopic surgical apparatus according to claim 1, wherein the treatment accessory (201; 301) includes a robotic accessory (201) with arms, a joint portion (203) connecting the arms to be movable relatively, and a driving mechanism configured to actuate the joint portion (203) to drive the arms, and the detecting means (201, 205, 220) is configured to perform detection by detecting directly or indirectly an actuation of the joint portion (203) of the robotic accessory (201).

**6.** The endoscopic surgical apparatus according to claim 1, wherein the setting means (201, 205, 220, 233; 301, 305, 220, 233) is configured to set by processing an observation image obtained by the endoscope.

**7.** The endoscopic surgical apparatus according to claim 1, wherein the detecting means (201, 205, 220) is configured to perform detection by processing an observation image obtained by the endoscope (101).

**8.** The endoscopic surgical apparatus according to claim 1, further comprising:
a warning means (253) configured to generate a warning;
wherein the control means (220) is configured to make the warning means (253) generate a warning on the basis of the movement when the treatment accessory (201; 301) is moved toward the outside of the control range (R).

**9.** The endoscopic surgical apparatus according to claim 1, further comprising:
an operating means (425) configured to operate the movement function or the treatment function of the treatment accessory (201; 301);
wherein the control means (220) is configured to control the operating means (425) to make amount of operation force necessary for manipulation increased or to force the operating means to be stopped when the treatment accessory (201; 301) is moved toward the outside of the control range (R).

**Patentansprüche**

**1.** Endoskopische Operationsvorrichtung, umfassend:

ein Behandlungszubehör (201; 301), das dazu ausgelegt ist, durch einen Kanal eines Endoskops (101) eingeführt zu werden und daraus herauszuragen, das einen Behandlungsabschnitt mit einer Behandlungsfunktion umfasst und eine Bewegungsfunktion aufweist;
ein Einstellmittel (201, 205, 220, 233), das dazu ausgelegt ist, einzustellen:
entweder

- eine Referenzposition (O) als eine Referenz bei der Behandlung durch das Behandlungszubehör (201) und eine Referenzrichtung (D) als eine Richtung von der Referenzposition (O) in ein Mukosagewebe (151) und orthogonal zu der Oberfläche des Mukosagewebes (151), und einen Referenzabstand (L) bezogen auf die Referenzposition (O), und dazu ausgelegt ist, eine Region als einen hemisphärischen Steuerbereich (R) mit Punkten einzustellen, die einen Abstand von der Referenzposition (O) aufweisen, der der Referenzabstand (L) oder darunter ist, wobei die Oberfläche des Mukosagewebes (151) die flache Seite der Hemisphäre darstellt; oder

- eine Referenzposition (O) als eine Referenz bei der Behandlung durch das Behandlungszubehör (301), eine erste und eine zweite Referenzrichtung, wobei die erste Referenzrichtung eine Richtung parallel zu der Oberfläche des Mukosagewebes (151) eines Flächenteils nahe eines Teils ist, an dem das Ansteigen eines betroffenen Teils (3) beginnt, und die zweite Referenzrichtung eine Richtung senkrecht zu der Oberfläche des Mukosagewebes (151) und in Richtung einer Körperwandseite ist, und erste und zweite Referenzabstände (L1, L2) bezogen auf die Referenzposition (O) und eine durch die Referenzposition (O) durchgehende Referenzachse, und dazu ausgelegt ist, eine Region als einen kolumnaren Steuerbereich (R) einzustellen, der sich über den zweiten Referenzabstand (L2) von der Referenzposition (O) erstreckt, mit Punkten, die einen Abstand von der Referenzachse aufweisen, bei dem es sich um den ersten Referenzabstand (L1) oder darunter handelt;

ein Erfassungsmittel (201, 205, 220), das dazu ausgelegt ist, zu erfassen, ob das Behandlungszubehör (201; 301) in Richtung der Außenseite des Steuerbereichs (R) bewegt wird oder nicht; und

ein Steuermittel (220), das dazu ausgelegt ist, eine Bewegung des Behandlungszubehörs (201; 301) in Richtung der Außenseite des Steuerbereichs (R) in einer Referenzrichtung zu steuern, die verlangsamt oder gestoppt wird, oder eine Behandlungsfähigkeit des Behandlungsabschnitts, die verlangsamt oder gestoppt wird, wenn das Behandlungszubehör (201; 301) in Richtung der Außenseite des Steuerbereichs (R) in der Referenzrichtung bewegt wird,

wobei die Referenzrichtung:

die Referenzrichtung (D) von der Referenzposition (O) in ein Mukosagewebe (151) und orthogonal zu der Oberfläche des Mukosagewebes (151) ist, wenn der Steuerbereich (R) der hemisphärische Steuerbereich ist, oder

die Richtung des Abstands (L2) weg von dem Referenzpunkts (O) bzw. die Richtung des Abstands (L1) weg von der Referenzachse ist, wenn der Steuerbereich (R) der kolumnare Steuerbereich ist.

2. Endoskopische Operationsvorrichtung nach Anspruch 1, wobei das Einstellmittel (201, 205, 220, 233; 301, 305, 220, 233) dazu ausgelegt ist, eine Mehrzahl von Referenzrichtungen (D1, D2) einzustellen, und sich eine Variable der Verlangsamung oder des Stillstands der Bewegung des Behandlungszubehörs (201; 301) oder eine Variable der Verringerung oder des Stillstands der Behandlungsfähigkeit mit Bezug auf die Bewegung für die Mehrzahl an Referenzrichtungen (D1, D2) voneinander unterscheiden.

3. Endoskopische Operationsvorrichtung nach Anspruch 1, wobei das Einstellmittel (201, 205, 220, 233; 301, 305, 220, 233) ein Einstellzubehör (301) mit einem Einstellabschnitt (252; 281; 302) zum Einstellen der Referenzposition (O) umfasst und dazu ausgelegt ist, die Bezugsposition (O) an einer Position einzustellen, an der der Einstellabschnitt (252; 281; 302) platziert ist.

4. Endoskopische Operationsvorrichtung nach Anspruch 1, wobei das Behandlungszubehör (201; 301) ein Energiezubehör (202) umfasst und die Behandlungsfähigkeit eine Ausgabegröße oder einen Ausgabemodus umfasst.

5. Endoskopische Operationsvorrichtung nach Anspruch 1, wobei das Behandlungszubehör (201; 301) ein Roboterzubehör (201) mit Armen, einen Gelenkabschnitt (203), der die Arme verbindet, damit sie relativ beweglich sind, und einen Antriebsmechanismus umfasst, der dazu ausgelegt ist, den Gelenkabschnitt (203) zu betätigen, um die Arme anzutreiben, und

das Erfassungsmittel (201, 205, 220) dazu ausgelegt ist, die Erfassung durch direktes oder indirektes Erfassen einer Betätigung des Gelenkabschnitts (203) des Roboterzubehörs (201) durchzuführen.

6. Endoskopische Operationsvorrichtung nach Anspruch 1, wobei das Einstellmittel (201, 205, 220, 233; 301, 305, 220, 233) dazu ausgelegt ist, sich durch Verarbeiten eines von dem Endoskop erhaltenen Beobachtungsbilds einzustellen.

7. Endoskopische Operationsvorrichtung nach Anspruch 1, wobei das Erfassungsmittel (201, 205, 220) dazu ausgelegt ist, die Erfassung durch Verarbeiten eines von dem Endoskop (101) erhaltenen Beobachtungsbilds durchzuführen.

**8.** Endoskopische Operationsvorrichtung nach Anspruch 1, die ferner umfasst:
ein Warnmittel (253), das dazu ausgelegt ist, eine Warnung zu erzeugen;
wobei das Steuermittel (220) dazu ausgelegt ist, dafür zu sorgen, dass das Warnmittel (253) eine Warnung auf der Basis der Bewegung erzeugt, wenn das Behandlungszubehör (201; 301) in Richtung der Außenseite des Steuerbereichs (R) bewegt wird.

**9.** Endoskopische Operationsvorrichtung nach Anspruch 1, die ferner umfasst:
ein Bedienmittel (425), das dazu ausgelegt ist, die Bewegungsfunktion oder die Behandlungsfunktion des Behandlungszubehörs (201; 301) zu bedienen;
wobei das Steuermittel (220) dazu ausgelegt ist, das Bedienmittel (425) zu steuern, um den Betrag der für die Manipulation erforderlichen Bedienkraft zu erhöhen oder das Bedienmittel dazu zu zwingen, anzuhalten, wenn das Behandlungszubehör (201; 301) in Richtung der Außenseite des Steuerbereichs (R) bewegt wird.

**Revendications**

**1.** Appareil chirurgical endoscopique comprenant :

un accessoire de traitement (201 ; 301) configuré pour être inséré à travers et se projet à partir d'un canal d'un endoscope (101), comprenant une partie de traitement avec une fonction de traitement, et ayant une fonction de mouvement ; des moyens de réglage (201, 205, 220, 233) configurés pour définir :

- une position de référence (O) comme référence pour traitement par l'accessoire de traitement (201) et une direction de référence (D) comme direction allant de la position de référence (O) à un tissu de muqueuse (151) et orthogonale à la surface du tissu de muqueuse (151), et une distance de référence (L) par rapport à la position de référence (O), et configurés pour définir une région comme plage de commande hémisphérique (R) avec des points ayant une distance par rapport à la position de référence (O) qui est la distance de référence (L) ou moins, la surface du tissu de muqueuse (151) représentant le côté plat de l'hémisphère ; ou
- une position de référence (O) comme référence pour traitement par l'accessoire de traitement (301), des première et seconde directions de référence, la première direction de référence étant une direction parallèle à la surface du tissu de muqueuse (151) d'une partie de plan à proximité d'une partie à partir de laquelle le soulèvement d'une partie affectée (3) est commencé, et la seconde direction de référence étant une direction perpendiculaire à la surface du tissu de muqueuse (151) et dirigée vers un côté paroi corporelle, et des première et seconde distances de référence (L1, L2) par rapport à la position de référence (O) et un axe de référence passant par la position de référence (O), et configurés pour définir une région comme plage de commande en colonne (R) qui s'étend sur la seconde distance de référence (L2) à partir de la position de référence (O) avec des points ayant une distance par rapport à l'axe de référence qui est la première distance de référence (L1) ou moins ;

des moyens de détection (201, 205, 220) configurés pour détecter si l'accessoire de traitement (201 ; 301) est déplacé vers l'extérieur de la plage de commande (R) ou non ; et
des moyens de commande (220) configurés pour commander un mouvement de l'accessoire de traitement (201 ; 301) vers l'extérieur de la plage de commande (R) dans une direction de référence, qui est amené à être ralenti ou arrêté, ou une capacité de traitement de la partie de traitement, qui est amenée à être réduite ou arrêtée, lorsque l'accessoire de traitement (201 ; 301) est déplacé vers l'extérieur de la plage de commande (R) dans la direction de référence,

la direction de référence étant :

la direction de référence (D) allant de la position de référence (O) à un tissu de muqueuse (151) et orthogonale à la surface du tissu de muqueuse (151), lorsque la plage de commande (R) est la plage de commande hémisphérique, ou
la direction de distance (L2) s'éloignant du point de référence (O), respectivement la direction de distance (L1) s'éloignant de l'axe de référence, lorsque la plage de commande (R) est la plage de commande en colonne.

**2.** Appareil chirurgical endoscopique selon la revendication 1, dans lequel les moyens de réglage (201, 205, 220, 233 ; 301, 305, 220, 233) sont configurés pour définir une pluralité de directions de référence (D1, D2), et une variable de ralentissement ou d'arrêt du mouvement de l'accessoire de traitement (201 ; 301) ou une variable de réduction ou d'arrêt de la capacité de traitement concernant le mouvement pour la pluralité de directions de référence (D1, D2) sont différentes l'une de l'autre.

3. Appareil chirurgical endoscopique selon la revendication 1, dans lequel les moyens de réglage (201, 205, 220, 233 ; 301, 305, 220, 233) comprennent un accessoire de réglage (301) avec une partie de réglage (252 ; 281 ; 302) pour définir la position de référence (O) et sont configurés pour définir la position de référence (O) sur une position où est placée la partie de réglage (252 ; 281 ; 302).

4. Appareil chirurgical endoscopique selon la revendication 1, dans lequel l'accessoire de traitement (201 ; 301) comprend un accessoire d'énergie (202) et la capacité de traitement comprend une grandeur de sortie ou un mode de sortie.

5. Appareil chirurgical endoscopique selon la revendication 1, dans lequel l'accessoire de traitement (201 ; 301) comprend un accessoire robotique (201) avec des bras, une partie articulation (203) reliant les bras pour qu'ils soient mobiles de manière relative, et un mécanisme d'entraînement configuré pour actionner la partie articulation (203) afin d'entraîner les bras, et
les moyens de détection (201, 205, 220) sont configurés pour réaliser une détection par détection directe ou indirecte d'un actionnement de la partie articulation (203) de l'accessoire robotique (201).

6. Appareil chirurgical endoscopique selon la revendication 1, dans lequel les moyens de réglage (201, 205, 220, 233 ; 301, 305, 220, 233) sont configurés pour définir par traitement d'une image d'observation obtenue par l'endoscope.

7. Appareil chirurgical endoscopique selon la revendication 1, dans lequel les moyens de détection (201, 205, 220) sont configurés pour réaliser une détection par traitement d'une image d'observation obtenue par l'endoscope (101).

8. Appareil chirurgical endoscopique selon la revendication 1, comprenant en outre :

   des moyens d'avertissement (253) configurés pour générer un avertissement ;
   les moyens de commande (220) étant configurés pour amener les moyens d'avertissement (253) à générer un avertissement sur la base du mouvement lorsque l'accessoire de traitement (201 ; 301) est déplacé vers l'extérieur de la plage de commande (R).

9. Appareil chirurgical endoscopique selon la revendication 1, comprenant en outre :

   des moyens d'actionnement (425) configurés pour actionner la fonction de mouvement ou la fonction de traitement de l'accessoire de traitement (201 ; 301) ;
   les moyens de commande (220) étant configurés pour commander les moyens d'actionnement (425) afin d'augmenter la grandeur de force d'actionnement nécessaire pour une manipulation ou de forcer l'arrêt des moyens d'actionnement lorsque l'accessoire de traitement (201 ; 301) est déplacé vers l'extérieur de la plage de commande (R).

FIG.1

233 — Function control input apparatus

225 — Joystick

D104

D100

211 — High frequency electric knife power apparatus

D106

D109

Robotic accessory control apparatus

220

D101

D105

205 — Motor box

D108

Robotic accessory

201

F I G. 2

F I G. 3

EP 2 108 327 B1

# FIG. 4

EP 2 108 327 B1

```
┌──────────────────────────────────────────────┐
│      Movement function control is selected     │──S1
└──────────────────────────────────────────────┘
                        │
                        ▼
┌──────────────────────────────────────────────┐
│         Reference point is set on position     │──S2
│          of distal end of robotic accessory    │
└──────────────────────────────────────────────┘
                        │
                        ▼
┌──────────────────────────────────────────────┐
│          Reference direction and reference     │──S3
│      distance relative to reference point is set│
└──────────────────────────────────────────────┘
                        │
                        ▼
┌──────────────────────────────────────────────┐
│ Control range of distal end of robotic accessory is │──S4
│ set wherein movement function control is performed │
└──────────────────────────────────────────────┘
                        │
                        ▼
┌──────────────────────────────────────────────┐
│      Movement of distal end of robotic accessory │──S5
└──────────────────────────────────────────────┘
                        │
                        ▼
                       S6
              ◇ Is distal end of                    ┌────────────────────────┐
            robotic accessory moved toward ─── Yes ─▶│  Velocity of movement of │  S7
              outside of control range              │   distal end of robotic  │
                       ?                            │  accessory toward outside│
                      No                            │  of control range is dropped│
                       │◀───────────────────────────│   or movement is stopped  │
                       ▼                            └────────────────────────┘
                       S8
              ◇ Is another setting of
            reference direction or reference distance ── Yes ──▶
              relative to reference point
                    selected?
                      No
                       │
                       ▼
                       S9
              ◇ Is another
            setting of reference point selected ── Yes ──▶
                       ?
                      No
                       │
                       ▼
                      S10
              ◇ Is release of
            movement function control selected ── No ──▶
                       ?
                      Yes
                       │
                       ▼
┌──────────────────────────────────────────────┐
│            Return to state where               │──S11
│    movement function control is not performed  │
└──────────────────────────────────────────────┘
```

FIG. 5

Treatment function control is selected —S21

Reference point is set on position of distal end of robotic accessory —S22

Reference direction and reference distance relative to reference point is set —S23

Control range of distal end of robotic accessory is set wherein treatment function control is performed —S24

Movement of distal end of robotic accessory —S25

S26 Is distal end of robotic accessory moved toward outside of control range ? — Yes → S27 Output of high frequency electric knife is decreased more or output is stopped as moved toward outside more

No

S28 Is another setting of reference direction or reference distance relative to reference point selected ? — Yes

No

S29 Is another setting of reference point selected ? — Yes

No

S30 Is release of treatment function control selected? — No

Yes

Return to state where treatment function control is not performed —S31

F I G. 6

FIG.7

FIG.8

FIG.9

FIG.10

FIG.11

FIG.12

α1(L1s)

1

0
0                                                    L

L1s

F I G. 13

α2(L2s)

1

0
0                                                    L

L2s

F I G. 14

F I G. 15

F I G. 16

CPU  245
Memory  246
247
Motor drive

233
231
241
230a  230d  230e  230b  230c

220
242

324  322
305
319
323

224  222
223  205
308  208  219

211
213  W
218
214a  214b
215
212

325
225

217

204
101

104
103

**Outside of body**

**Inside of body**

301
303
302
203
3  201
202

216

F I G. 17

EP 2 108 327 B1

35

F I G. 18

EP 2 108 327 B1

FIG.19

| Movement function control is selected | —S41 |

Reference point is set on position of distal end of second robotic accessory — S42

Reference direction and reference distance relative to reference point is set — S43

Control range of distal end of first robotic accessory is set wherein movement function control is performed — S44

Movement of distal end of first robotic accessory — S45

**S46** Is distal end of first robotic accessory moved toward outside of control range? — **Yes** → **S47** Velocity of movement of distal end of first robotic accessory toward outside of control range is dropped or movement is stopped

**No**

**S48** Is another setting of reference direction or reference distance relative to reference point selected? — **Yes**

**No**

**S49** Is another setting of reference point selected? — **Yes**

**No**

**S50** Is release of movement function control selected? — **No**

**Yes**

Return to state where movement function control is not performed — S51

# F I G. 20

38

F I G. 21

EP 2 108 327 B1

F I G. 22

FIG.23

F I G. 24

F I G. 25

245 246 253

CPU   Memory

220

231
233
230b
241

Motor
drive

247
242

230a

230d  230e  254  224  222

223  205

208

219

211  213

W

218
215

214a  214b  212  204  101

217

Outside of body

Inside of body

201

3  202  203

216

104

103

225

EP 2 108 327 B1

43

Warning control is selected ⎯ S61

Reference point is set on position of distal end of robotic accessory ⎯ S62

Reference direction and reference distance relative to reference point is set ⎯ S63

Control range of distal end of robotic accessory is set wherein warning control is performed ⎯ S64

Movement of distal end of robotic accessory ⎯ S65

S66
Is distal end of robotic accessory moved toward outside of control range ? — Yes →

S67
Sound volume of warning sound is turned up more as distal end of robotic accessory is move toward outside more

No

S68
Is another setting of reference direction or reference distance relative to reference point selected ? — Yes

No

S69
Is another setting of reference point selected ? — Yes

No

S70
Is release of warning control selected ? — No

Yes

Return to state where warning control is not performed ⎯ S71

F I G. 26

F I G. 27

Active joystick ~425

233~ Function control input apparatus

Motor box ~256

D104

211

High frequency electric knife power apparatus

D106

D109

Robotic accessory control apparatus

D115  D116

D101

205

Motor box

D105

220

D108

Robotic accessory

201

F I G. 28

**Active control is selecetd** — S81

↓

**Reference point is set on position of distal end of robotic accessory** — S82

↓

**Reference direction and reference distance relative to reference point is set** — S83

↓

**Control range of distal end of robotic accessory is set wherein active control is performed** — S84

↓

**Movement of distal end of robotic accessory** — S85

↓

**S86** Is distal end of robotic accessory moved toward outside of control range? —Yes→ **S87** Amount of force necessary for manipulating active joystick is increased more or joystick is stopped as moved toward outside of control range more

No ↓

**S88** Is another setting of reference direction or reference distance relative to reference point selected ? —Yes→

No ↓

**S89** Is another setting of reference point selected ? —Yes→

No ↓

**S90** Is release of active control selected ? —No→

Yes ↓

**Return to state where active control is not performed** — S91

F I G. 29

F I G. 30

F I G. 31

EP 2 108 327 B1

```
┌─────────────────────────────┐
│ Following control is selected │──S100
└─────────────────────────────┘
                │
                ▼
┌─────────────────────────────┐
│ Following condition is set    │──S101
└─────────────────────────────┘
                │
                ▼
┌─────────────────────────────┐
│ Movement of second          │──S102
│ robotic accessory           │
└─────────────────────────────┘
                │
                ▼
┌─────────────────────────────┐
│ Following movement of       │──S103
│ first robotic accessory     │
└─────────────────────────────┘
                │
                ▼
              S104
         ╱─────────╲
        ╱  Is another ╲     Yes
       ╱ setting       ╲──────
       ╲ of following   ╱
        ╲ condition    ╱
         ╲ selected? ╱
          ╲───────╱
              │ No
              ▼
              S105
         ╱─────────╲
        ╱  Is release ╲     No
       ╱ of following  ╲──────
       ╲ control       ╱
        ╲ selected?   ╱
          ╲───────╱
              │ Yes
              ▼
┌─────────────────────────────┐
│ Return to state where       │──S106
│ following control is not performed │
└─────────────────────────────┘
```

F I G. 32

F I G. 33

Automatic movement control is selected —S120

Reference point is set on position of
distal end of second robotic accessory —S121

Start of automatic movement control is selected —S122

Distal end of first robotic accessory is
automatically moved from
present position toward reference point —S123

S124

Is release
of automatic movement control
selected?

Yes

No

Distal end of first robotic accessory
is moved to reference point —S125

Return to state where
automatic movement control is not performed —S126

F I G. 34

**REFERENCES CITED IN THE DESCRIPTION**

**Patent documents cited in the description**

- JP 5042167 A
- JP 2004223128 A
- JP 9131351 A
- US 20060142657 A1